(19) Europäisches Patentamt
European Patent Office
Office européen des brevets

(11) EP 1 202 953 B1

(12) # EUROPÄISCHE PATENTSCHRIFT

(45) Veröffentlichungstag und Bekanntmachung des Hinweises auf die Patenterteilung:
**30.08.2006 Patentblatt 2006/35**

(21) Anmeldenummer: **00951440.7**

(22) Anmeldetag: **25.07.2000**

(51) Int Cl.:
*C07C 211/27* (2006.01) *C07C 215/50* (2006.01)
*C07C 217/58* (2006.01) *A61K 31/135* (2006.01)
*C07D 307/79* (2006.01) *C07D 317/58* (2006.01)
*C07C 213/02* (2006.01) *C07C 209/68* (2006.01)

(86) Internationale Anmeldenummer:
**PCT/EP2000/007095**

(87) Internationale Veröffentlichungsnummer:
**WO 2001/010816 (15.02.2001 Gazette 2001/07)**

(54) **SUBSTITUIERTE 2-DIALKYLAMINOALKYLBIPHENYL-DERIVATE**
SUBSTITUTED 2-DIALKYLAMINOALKYLBIPHENYL DERIVATIVES
DERIVES DE 2-DIALKYLAMINOALKYLBIPHENYLE

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **09.08.1999 DE 19937537**

(43) Veröffentlichungstag der Anmeldung:
**08.05.2002 Patentblatt 2002/19**

(73) Patentinhaber: **Grünenthal GmbH
52078 Aachen (DE)**

(72) Erfinder:
- **SUNDERMANN, Bernd
D-52070 Aachen (DE)**
- **KÖGEL, Babette-Yvonne
D-52379 Langerwehe-Hamich (DE)**
- **BUSCHMANN, Helmut
D-52066 Aachen (DE)**

(56) Entgegenhaltungen:
**US-A- 3 443 943** **US-A- 4 473 709**
**US-A- 5 672 596**

- **A. MISIORNY ET AL.: "Chemistry and CNS-effects of 2-aminomethyl-2'-hyfdroxybiphenyls and 2-aminomethyl-2'-3'-dihydroxybiphenyls" ACTA PHARMACEUTICA SUECICA., Bd. 14, 1977, Seiten 105-112, XP000926011 ISSN: 0001-6675**
- **SHIGERU KOBAYASHI ET AL.: "Syntheses of Apogalanthamine analogs as alpha-adrenergic blocking agents" CHEMICAL AND PHARMACEUTICAL BULLETIN., Bd. 25, 1977, Seiten 3312-3323, XP000952775 PHARMACEUTICAL SOCIETY OF JAPAN. TOKYO., JP ISSN: 0009-2363**
- **SHIGERU KOBAYASHI ET AL.: "The constitution of apoGalanthamine" JOURNAL OF THE CHEMICAL SOCIETY., 1957, Seiten 638-645, XP000952772 CHEMICAL SOCIETY. LETCHWORTH., GB**
- **ROBERT J. DE VITA ET AL.: "A potent, orally bioavailable benzazepinone growth hormone secretagogue" JOURNAL OF MEDICINAL CHEMISTRY., Bd. 41, Nr. 10, 1998, Seiten 1716-1728, XP000926002 WASHINGTON US**
- **MOSHE WETZBERG ET AL.: "Syntheses and chemistry of some dibenz(c,e)azepines" JOURNAL OF ORGANIC CHEMISTRY., Bd. 52, Nr. 4, 1987, Seiten 529-536, XP002150438 EASTON US**
- **P. W. JEFFS ET AL.: "Biosynthesis of mesembrine and related alkaloids." JOURNAL OF THE AMERICAN CHEMICAL SOCIETY., Bd. 93, Nr. 15, 1971, Seiten 3752-3758, XP002150439 DC US**
- **KEVIN E. CULLEN ET AL.: "Reactions of diene-conjugated 1,3-dipolar intermediates:" JOURNAL OF THE CHEMICAL SOCIETY, PERKIN TRANSACTIONS 1., 1993, Seiten 2961-2967, XP002150440 LETCHWORTH GB**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 202 953 B1

- CHEMICAL ABSTRACTS, vol. 84, no. 9, 1. März 1976 (1976-03-01) Columbus, Ohio, US; abstract no. 58806t, BABAYAN, A. T. ET AL.: "Intramolecular rearrangement of ammonium yilde formed by the splitting of substituted dihydroisoindolinium salts" Seite 461; Spalte 2; XP002150443 & DOKL. AKAD. NAUK ARM. SSR, Bd. 61, Nr. 1, 1975, Seiten 40-43,
- HANS-G. BOIT: "Beilsteins Handbuch der organischen Chemie, Band XII, vierte Auflage, drittes Ergänzungswerk, Seite 3235, 3252" 1973 , SPRINGER-VERLAG , BERLIN . HEIDELBERG . NEW YORK XP002150441 Seite 3235, Absätze 6-9; Seite 3252, Absätze 5-7
- REINER LUCKENBACH: "Beilsteins Handbuch der organischen Chemie, Band XII, vierte Auflage, viertes Ergänzungswerk, Seite 3329" 1984 , SPRINGER-VERLAG , BERLIN . HEIDELBERG . NEW YORK . TOKYO XP002150442 Seite 3329, Absatz 3
- H.-W. BERSCH ET AL.: "o-(o-(Dimethylaminomethyl)phenyl)-benzaldehyd." ARCHIV DER PHARMAZIE., Bd. 291, 1958, Seiten 91-94, XP000926009 VCH VERLAGSGESELLSCHAFT MBH, WEINHEIM., DE ISSN: 0365-6233
- H.-W. BERSCH ET AL.: "Phenanthrenbildung durch Hofmann-Abbau" ARCHIV DER PHARMAZIE., Bd. 290, 1957, Seiten 353-357, XP000926010 VCH VERLAGSGESELLSCHAFT MBH, WEINHEIM., DE ISSN: 0365-6233
- HARRY FINCH ET AL.: "An efficient general route to Furo-, Pyrido- and thieno-(d)(2) benzazepines..." JOURNAL OF THE CHEMICAL SOCIETY, PERKIN TRANSACTIONS 1., 1994, Seiten 1193-1203, XP002159779 LETCHWORTH GB

## Beschreibung

Die Erfindung betrifft substituierte 2-Aminoalkylbiphenyl-Derivate, Verfahren zu deren Herstellung, Arzneimittel enthaltend diese Verbindungen, sowie die Verwendung dieser Verbindungen zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie z.B. Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation, Sucht, Abhängigkeit und Toleranzentwicklung limitiert. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990). Außerdem zeigen sie bei einigen Schmerzzuständen, insbesondere bei neuropathischen Schmerzen, eine geringere Wirksamkeit.

Aus den Veröffentlichungen von A. Misiorny et al., Acta Pharm: Suec. Bd. 14, 1977, Seiten 105-112; S. Kobayashi et al., Chemical and Pharmaceutical Bulletin, Bd. 25, 1977, Seiten 3312-3323; S. Kobayashi et al., Journal of the Chemical Society, 1957, Seiten 638-645; M. Wetzberg et al. Journal of Organic Chemistry, Bd. 52, Nr. 4, 1987, Seiten 529-536; P.W. Jeffs et al., Journal of the American Chemical Society, Bd. 93, Nr. 15, 1971, Seiten 3752-3758; Kevin E. Cullen et al., Journal of the Chemical Society, Perkin Transactions 1., 1993, Seiten 2961-2967; A.T. Babayan et al., Dokl. Akad. Nauk. Arm. Ssr., Bd. 61, Nr. 1, 1975, Seiten 40-43; Hans G. Boit, Beilsteins Handbuch der organischen Chemie, Band XII, vierte Auflage, drittes Ergänzungswerk, Seite 3235, 3252', 1973; Reiner Luckenbach, Beilsteins Handbuch der organischen Chemie, Band XII, vierte Auflage, viertes Ergänzungswerk, Seite 3329'; Robert J. de Vita et al., Journal of Medicinal Chemistry, Bd. 41, Nr. 10, 1998, Seiten 1716-1728; H.W. Bersch et al., Archiv der Pharmazie, Bd. 291, 1958, Seiten 91-94 sowie aus US 3,443,943, US 4,473,709 und WO 98/57951 sind verschiedene substituierte Aminoalkylbiphenyl-Derivate bekannt.

Eine der Erfindung zugrundeliegende Aufgabe bestand darin, analgetisch wirksame Substanzen zur Verfügung zu stellen, die sich zur Schmerztherapie eignen. Darüber hinaus sollten diese Substanzen möglichst wenig Nebenwirkungen der Opioid-Analgetika wie z.B. Übelkeit, Erbrechen, Abhängigkeit, Atemdepression oder Obstipation aufweisen. Weitere Aufgaben bestanden darin, Wirkstoffe zur Behandlung von inflammatorischen und allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen und/oder Epilepsie zur Verfügung zu stellen.

Erfindungsgemäß wird dies durch das zur Verfügung stellen von neuen substituierten 2-Aminoalkylbiphenyl-Derivaten erreicht, die sich zur Behandlung von inflammatorischen und allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen und/oder Epilepsie eignen und die außerdem eine ausgeprägte analgetische Wirkung aufweisen

Gegenstand der Erfindung sind daher substituierte 2-Aminoalkyl-biphenyl-Derivate der allgemeinen Formel I,

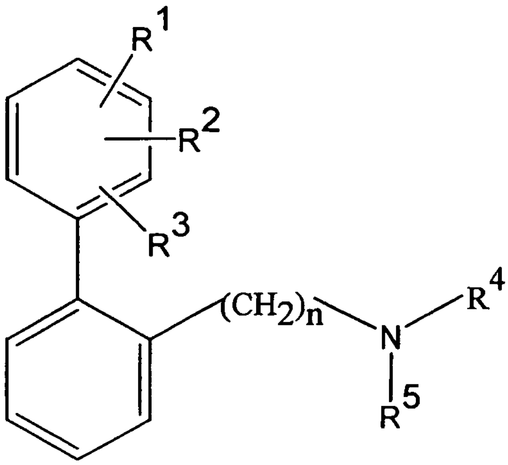

I

worin

n = 1 oder 2 ist,

der Rest $R^1$ für CN, $NO_2$, $SO_2CH_3$, $SO_2CF_3$, O-Aryl, O-$C_{1-6}$-Alkylen-Aryl, $NR^6R^7$, einen Aryl-, einen Acetyl, einen Acetamidyl-, oder für einen über eine $C_{1-6}$-AlkylenGruppe gebundenen, vorzugsweise für einen über eine $C_{1-3}$-Alkylen-Gruppe gebundenen Aryl-Rest steht,

der Rest $R^2$ für H, F, Cl, Br, CN, $NO_2$, CHO, $SO_2CH_3$, $SO_2CF_3$, $OR^6$, $NR^6R^7$, einen $C_{1-6}$-Alkyl-, vorzugsweise einen $C_{1-3}$-Alkyl-, einen Aryl-, einen Acetyl, einen Acetamidyl-, einen Benzoyl- oder für einen über eine $C_{1-6}$-Alkylen-Gruppe gebundenen, vorzugsweise für einen über eine $C_{1-3}$-Alkylen-Gruppe gebundenen Aryl-Rest steht,

oder $R^1$ und $R^2$ zusammen jeweils die Gruppe $OCH_2CH_2O$, CH=CHO, $CH=C(CH_3)O$ oder CH=CHNH bedeuten,

der Rest $R^3$ für H, F, Cl, Br, CN, $NO_2$, CHO, $SO_2CH_3$, $SO_2CF_3$, $OR^6$, $NR^6R^7$, einen $C_{1-6}$-Alkyl-, , vorzugsweise einen $C_{1-3}$-Alkyl-, einen Aryl-, einen Acetyl, einen Acetamidyl-, einen Benzoyl- oder für einen über eine $C_{1-6}$-Alkylen-Gruppe gebundenen, vorzugsweise für einen über eine $C_{1-3}$-Alkylen-Gruppe gebundenen Aryl-Rest steht,

die Reste $R^4$, $R^5$, gleich oder verschieden, für H oder für einen $C_{1-6}$-Alkyl-, vorzugsweise für einen $C_{1-3}$-Alkyl-Rest stehen,

die Reste $R^6$, $R^7$, gleich oder verschieden, für H, einen $C_{1-6}$-Alkyl-, vorzugsweise einen $C_{1-3}$-Alkyl-, einen Aryl- oder für einen über eine $C_{1-6}$-Alkylen-Gruppe gebundenen, vorzugsweise für einen über eine $C_{1-3}$-Alkylen-Gruppe gebundenen Aryl-Rest stehen,

in Form ihrer Basen und/oder Salze von physiologisch verträglichen Säuren, wobei die Verbindungen

4-Chlor-2'-dimethylaminomethylbiphenyl-2-carbonitril und

4-(2'-N,N-dimethylaminomethylphenyl)-2-fluoranilin

ausgenommen sind.

Unter Alkyl-Resten werden auch mindestens einfach, vorzugsweise mit Halogen und/oder einer Hydroxy-Gruppe, besonders bevorzugt mit Fluor und/oder einer Hydroxygruppe substituierte Kohlenwasserstoffe verstanden. Enthalten diese mehr als einen Substituenten, so können diese gleich oder verschieden sein. Vorzugsweise sind die Alkyl-Reste Methyl, Ethyl, Propyl, 1-Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, $CHF_2$, $CF_3$ oder $CH_2OH$.

Unter einem Aryl-Rest werden auch mindestens einfach mit einem OH-, einem Halogen-, vorzugsweise F- und/oder Cl-, einem $CF_3$-, einem $C_{1-6}$-Alkyl-, einem $C_{1-6}$-Alkoxy-, einem $C_{1-7}$-Cycloalkoxy-, einem $C_{3-7}$-Cycloalkyl-, einem $C_{2-6}$-Alkylen- oder Phenyl-Rest substituierte Phenyle oder Naphtyl-Reste verstanden. Die Phenylreste können auch mit weiteren Ringen kondensiert sein.

Besonders bevorzugt sind folgende substituierte 2-Aminoalkyl-biphenyl-Derivate:

Dimethyl-[2-(2-methylbenzofuran-4-yl)benzyl]amin und das entsprechende Hydrochlorid

Dimethyl-(4'-nitro-3'-trifluormethylbiphenyl-2-ylmethyl)-amin und das entsprechende Hydrochlorid

N-(2'-Dimethylaminomethyl-3-trifluormethoxybiphenyl-4-yl)acetamid und das entsprechende Hydrochlorid

[2-(1*H*-Indol-5-yl)benzyl]dimethylamin und das entsprechende Hydrochlorid

(4'-Methansulfonylbiphenyl-2-ylmethyl)dimethylamin und das entsprechende Hydrochlorid

1-[2'-(2-Dimethylaminoethyl)biphenyl-3-yl]ethanon und das entsprechende Hydrochlorid

Dimethyl-(3'-nitrobiphenyl-2-ylmethyl)amin und das entsprechende Hydrochlorid

4-Amino-2'-dimethylaminomethylbiphenyl-3-ol und das entsprechende Dihydrochlorid

2'-Dimethylaminomethyl-5-trifluormethoxybiphenyl-2-ylamin und das entsprechende Dihydrochlorid

N-(2'-Dimethylaminomethyl-5-trifluormethoxybiphenyl-2-yl)acetamid und das entsprechende Hydrochlorid

3,5-Dichlor-2'-dimethylaminomethyl-biphenyl-4-ylamin und das entsprechende Hydrochlorid.

Ein weiterer Gegenstand der Erfindung sind Verfahren zur Herstellung von substituierten 2-Aminoalkylbiphenyl-Derivaten der allgemeinen Formel I, welche dadurch gekennzeichnet sind, daß man die Verbindungen der allgemeinen Formel II, worin Y = Cl, Br oder I und m = 0 oder 1 bedeuten,

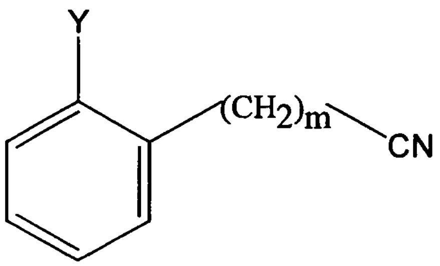

II

in Lösung mit einem Reduktionsmittel, vorzugsweise Lithiumaluminiumhydrid und/oder Diisobutylaluminiumhydrid, zu Verbindungen der allgemeinen Formel III, worin n = 1 oder 2 bedeutet, reduziert

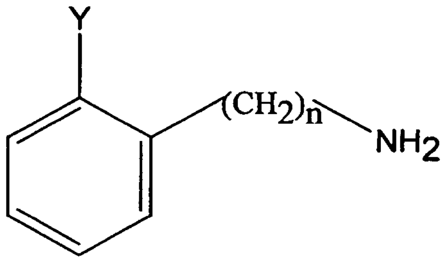

III

und diese nach üblichen Methoden reinigt und isoliert.

Die Verbindungen der allgemeinen Formel III werden in Gegenwart eines Reduktionsmittels, vorzugsweise Ameisensäure und/oder Natriumborhydrid mit aliphatischen $C_{1-6}$-Aldehyden zu Verbindungen der allgemeinen Formel IV umgesetzt,

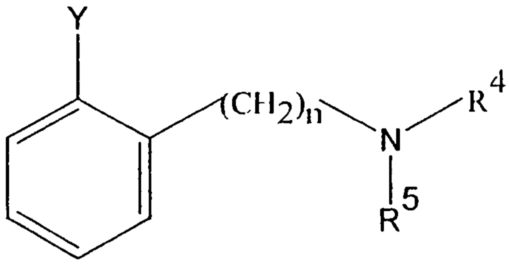

IV

worin $R^4$ und $R^5$ die Bedeutung gemäß der allgemeinen Formel haben, und nach den üblichen Methoden gereinigt und isoliert.

Die Verbindungen der allgemeinen Formel IV werden durch Halogen-Metallaustausch, vorzugsweise mit Magnesium und/oder Butyllithium und anschließender Reaktion mit einem Borsäureester, vorzugsweise einem Trialkylborat, besonders bevorzugt mit einem Trimethylborat bei Temperaturen ≤ 0 °C zu Verbindungen der allgemeinen Formel V, worin R einen $C_{1-6}$-Alkyl-Rest bedeutet, umgesetzt,

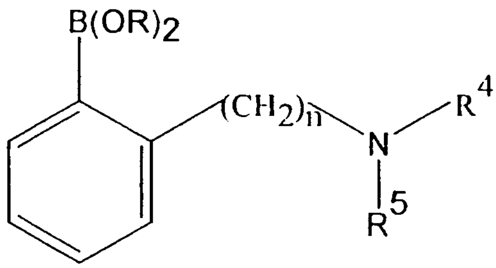

V

und nach üblichen Methoden isoliert und gereinigt.

Die Verbindungen der allgemeinen Formel V können mit wäßrigen Säuren, vorzugsweise Salzsäure, zu Verbindungen der allgemeinen Formel VI umgesetzt,

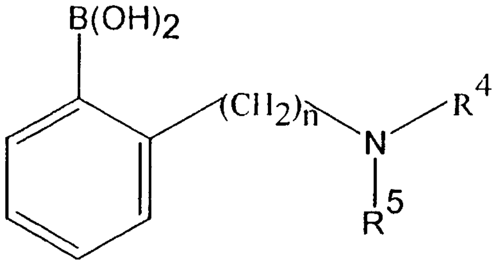

VI

und nach den üblichen Methoden gereinigt und isoliert werden.

Die Verbindungen der allgemeinen Formel V oder VI werden in einer Übergangsmetall-katalysierten Reaktion, vorzugsweise in einer durch Palladium(0)-Verbindungen oder durch Palladium(II)-Salze, besonders bevorzugt durch Tetrakis(triphenylphosphin)palladium, Bis(dibenzylidenaceton)palladium, elementares Palladium auf Aktivkohle, Palladium(II)chlorid und/oder Palladium(11)acetat katalysierten Reaktion in einem aliphatischen Ether, vorzugsweise 1,4-Dioxan und Tetrahydrofuran oder einem Kohlenwasserstoff, vorzugsweise Toluol oder Hexan, einem Alkohol, vorzugsweise Ethanol oder Isopropanol, einem chlorierten Kohlenwasserstoff, vorzugsweise Chloroform oder Dichlormethan in Wasser oder Gemischen aus diesen Lösungsmitteln bei Temperaturen zwischen 20 und 150 °C mit Verbindungen der allgemeinen Formel VII.

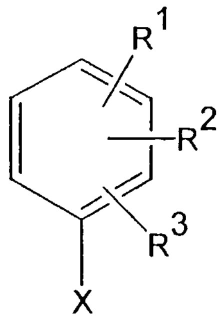

VII,

worin X = Cl, Br, I oder $OSO_2CpF_{(2+1)}$ bedeutet und die Reste $R^1$ bis $R^3$ die Bedeutung gemäß der allgemeinen Formel 1 haben, zu Verbindungen der allgemeinen Formel umgesetzt und nach den üblichen Methoden gereinigt und isoliert.

Alternativ dazu werden die Verbindungen der allgemeinen Formel VIII oder IX

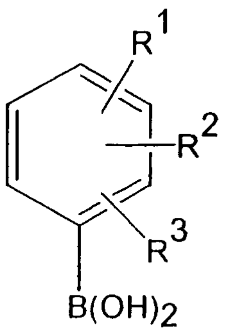

VIII

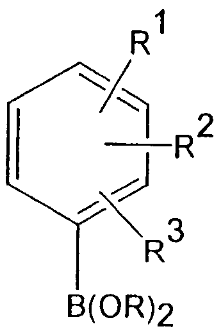

IX,

worin $R^1$ bis $R^3$ die Bedeutung gemäß der allgemeinen Formel 1 haben, in einer Übergangsmetall-katalysierten Reaktion, vorzugsweise in einer durch Palladium(0)-Verbindungen oder durch Palladium(II)-Salze, besonders bevorzugt durch

Tetrakis(triphenylphosphin)palladium, Bis(dibenzylidenaceton)palladium, elementares Palladium auf Aktivkohle, Palladium(II)chlorid und/oder Palladium(II)acetat katalysierten Reaktion in einem aliphatischen Ether, vorzugsweise 1,4-Dioxan und Tetrahydrofuran oder einem Kohlenwasserstoff, vorzugsweise Toluol oder Hexan, einem Alkohol, vorzugsweise Ethanol oder Isopropanol, einem chlorierten Kohlenwasserstoff, vorzugsweise Chloroform oder Dichlormethan in Wasser oder Gemischen aus diesen Lösungsmitteln bei Temperaturen zwischen 20 und 150 °C mit Verbindungen der allgemeinen Formel III oder IV zu Verbindungen der allgemeinen Formel I umgesetzt und nach den üblichen Methoden gereinigt und isoliert.

Die Verbindungen der allgemeinen Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in der an sich bekannten Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in wäßriger Lösung.

Die erfindungsgemäßen bzw. erfindungsgemäß zum Einsatz kommenden substituierten 2-Aminoalkylbiphenyl-Derivate der allgemeinen Formel I sind toxikologisch unbedenklich und stellen daher geeignete pharmazeutische Wirkstoffe dar.

Ein weiterer Gegenstand der Erfindung sind daher Arzneimittel, die als Wirkstoff wenigstens ein substituiertes 2-Aminoalkylbiphenyl-Derivat der allgemeinen Formel I enthalten,

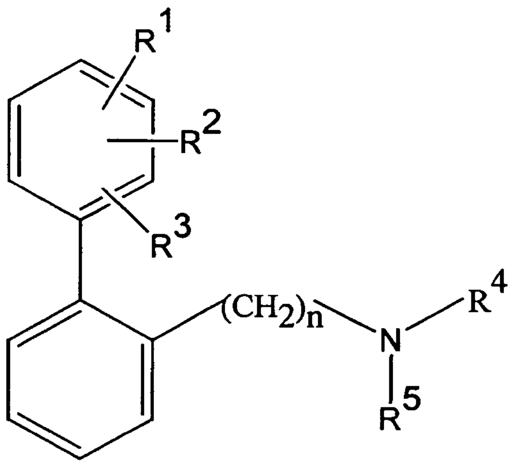

I,

worin

n = 1 oder 2 ist,

die Reste $R^1$, $R^2$, $R^3$, gleich oder verschieden für H, F, Cl, Br, CN, $NO_2$, CHO, $SO_2CH_3$, $SO_2CF_3$, $OR^6$, $NR^6R^7$, einen $C_{1-6}$-Alkyl-, vorzugsweise einen $C_{1-3}$-Alkyl-, einen Aryl-, einen Acetyl, einen Acetamidyl-, einen Benzoyl- oder für einen über eine $C_{1-6}$-Alkylen-Gruppe gebundenen, vorzugsweise für einen über eine $C_{1-3}$-Alkylen-Gruppe gebundenen Aryl-Rest stehen,

oder $R^1$ und $R^2$ zusammen jeweils die Gruppe $OCH_2O$, $OCH_2CH_2O$, CH=CHO, $CH=C(CH_3)O$ oder CH=CHNH bedeuten,

die Reste $R^4$, $R^5$, gleich oder verschieden, für H oder für einen $C_{1-6}$-Alkyl-, vorzugsweise für einen $C_{1-3}$-Alkyl-Rest stehen,

die Reste $R^6$, $R^7$, gleich oder verschieden, für H, einen $C_{1-6}$-Alkyl-, vorzugsweise für einen $C_{1-3}$-Alkyl-, einen Aryl- oder für einen über eine $C_{1-6}$-Alkylen-Gruppe gebundenen, vorzugsweise für einen über eine $C_{1-3}$-Alkylen-Gruppe gebundenen Aryl-Rest stehen,

in Form seiner Base und/oder eines Salzes einer physiologisch verträglichen Säure und gegebenfalls weitere Wirkstoffe und/oder Hilfsstoffe.

Unter Alkyl-Resten werden auch mindestens einfach, vorzugsweise mit Halogen und/oder einer Hydroxy-Gruppe, besonders bevorzugt mit Fluor und/oder einer Hydroxygruppe substituierte Kohlenwasserstoffe verstanden. Enthalten diese mehr als einen Substituenten, so können diese gleich oder verschieden sein. Vorzugsweise sind die Alkyl-Reste Methyl, Ethyl, Propyl, 1-Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, $CHF_2$, $CF_3$ oder $CH_2OH$.

Unter einem Aryl-Rest werden auch mindestens einfach mit einem OH-, einem Halogen-, vorzugsweise F- und/oder Cl-, einem $CF_3$-, einem $C_{1-6}$-Alkyl-, einem $C_{1-6}$-Alkoxy-, einem $C_{1-7}$-Cycloalkoxy-, einem $C_{3-7}$-Cycloalkyl-, einem $C_{2-6}$-Alkylen- oder Phenyl-Rest substituierte Phenyle oder Naphtyl-Reste verstanden. Die Phenylreste können auch mit weiteren Ringen kondensiert sein.

Besonders bevorzugt ist ein Arzneimittel enthaltend als pharmazeutischen Wirkstoff wenigstens ein substituiertes 2-Aminoalkylbiphenyl-Derivat ausgewählt aus der Gruppe umfassend folgende Verbindungen:

(3'-Methoxybiphenyl-2-ylmethyl)dimethylamin und das entsprechende Hydrochlorid,

(4'-Chlorbiphenyl-2-ylmethyl)dimethylamin und das entsprechende Hydrochlorid,

2'-Dimethylaminomethylbiphenyl-3-ol und das entsprechende Hydrochlorid,

(2'-Methoxybiphenyl-2-ylmethyl)dimethylamin und das entsprechende Hydrochlorid,

(3'-Chlorbiphenyl-2-ylmethyl)dimethylamin und das entsprechende Hydrochlorid,

(2'-Fluorbiphenyl-2-ylmethyl)dimethylamin und das entsprechende Hydrochlorid,

(3'-Fluorbiphenyl-2-ylmethyl)dimethylamin und das entsprechende Hydrochlorid,

(4'-Fluorbiphenyl-2-ylmethyl)dimethylamin und das entsprechende Hydrochlorid,

(3'-Chlor-4'-fluorbiphenyl-2-ylmethyl)dimethylamin und das entsprechende Hydrochlorid,

(3'-Methoxybiphenyl-2-ylethyl)dimethylamin und das entsprechende Hydrochlorid,

Dimethyl-[2-(2-methylbenzofuran-4-yl)benzyl]amin und das entsprechende Hydrochlorid,

2'-Dimethylaminomethylbiphenyl-2-carbaldehyd Hydrochlorid,

(3'-Difluormethylbiphenyl-2-ylmethyl)dimethylamin und das entsprechende Hydrochlorid,

2'-Dimethylaminomethylbiphenyl-3-carbaldehyd und das entsprechende Hydrochlorid,

Biphenyl-2-ylmethyldimethylamin Hydrochlorid,

(3',4'-Dichlorbiphenyl-2-ylmethyl)dimethylamin und das entsprechende Hydrochlorid,

(3',5'-Dichlorbiphenyl-2-ylmethyl)dimethylamin und das entsprechende Hydrochlorid,

Dimethyl-(4'-nitro-3'-trifluormethylbiphenyl-2-ylmethyl)-amin und das entsprechende Hydrochlorid,

(3',4'-Difluorbiphenyl-2-ylmethyl)dimethylamin und das entsprechende Hydrochlorid,

(4'-Fluor-3'-trifluormethylbiphenyl-2-ylmethyl)dimethyl-amin und das entsprechende Hydrochlorid,

(4'-Chlor-3'-methoxybiphenyl-2-ylmethyl)dimethylamin und das entsprechende Hydrochlorid,

N-(2'-Dimethylaminomethyl-3-trifluormethoxybiphenyl-4-yl)acetamid und das entsprechende Hydrochlorid,

(3'-Isopropoxybiphenyl-2-ylmethyl)dimethylamin und das entsprechende und das entsprechende Hydrochlorid,

2'-(2-Dimethylaminoethyl)biphenyl-3-ol und das entsprechende Hydrochlorid,

4-Chlor-2'-dimethylaminomethylbiphenyl-3-ol und das entsprechende Hydrochlorid,

[2-(1*H*-Indol-5-yl)benzyl]dimethylamin und das entsprechende Hydrochlorid,

(4'-Methansulfonylbiphenyl-2-ylmethyl)dimethylamin und das entsprechende Hydrochlorid,

(2',4'-Dichlorbiphenyl-2-ylmethyl)dimethylamin und das entsprechende Hydrochlorid,

(2',3'-Difluorbiphenyl-2-ylmethyl)dimethylamin und das entsprechende Hydrochlorid,

(2',5'-Difluorbiphenyl-2-ylmethyl)dimethylamin und das entsprechende Hydrochlorid,

(2-Benzo[1,3]dioxol-5-ylbenzyl)dimethylamin und das entsprechende Hydrochlorid,

1-[2'-(2-Dimethylaminoethyl)biphenyl-3-yl]ethanon und das entsprechende Hydrochlorid,

[2-(3',4'-Dimethoxybiphenyl-2-ylethyl]dimethylamin und das entsprechende Hydrochlorid,

[2-(3'-lsopropoxybiphenyl-2-yl)ethyl]dimethylamin und das entsprechende Hydrochlorid,

[2-(4'-Chlor-3'-methoxybiphenyl-2-yl)ethyl]dimethylamin und das entsprechende Hydrochlorid,

4-Chlor-2'-(2-dimethylaminoethyl)biphenyl-3-ol und das entsprechende Hydrochlorid,

Dimethyl-(3'-nitrobiphenyl-2-ylmethyl)amin und das entsprechende Hydrochlorid,

4-Amino-2'-dimethylaminomethylbiphenyl-3-ol und das entsprechende Dihydrochlorid,

(3',5'-Difluorbiphenyl-2-ylmethyl)dimethylamin und das entsprechende Hydrochlorid,

(2',5'-Dimethoxybiphenyl-2-ylmethyl)dimethylamin und das entsprechende Hydrochlorid,

2'-Dimethylaminomethyl-5-trifluormethoxybiphenyl-2-ylamin und das entsprechende Dihydrochlorid,

N-(2'-Dimethylaminomethyl-5-trifluormethoxybiphenyl-2-yl)acetamid und das entsprechende Hydrochlorid sowie

3,5-Dichlor-2'-dimettiylaminomethyl-biphenyl-4-ylamin und das entsprechende Hydrochlorid.

Vorzugsweise werden die Arzneimittel zur Behandlung/Bekämpfung bei/von Schmerzen, inflammatorischen und allergischen Reaktionen, Depressionen, Drogen- und Alkoholmißbrauch, Gastritis, Diarrhoe, Harninkontinenz, cardiovaskulären Erkrankungen, Atemwegserkrankungen, Husten, seelischen Erkrankungen und/oder Epilepsie eingesetzt.

Ein weiterer Gegenstand der Erfindung ist auch die Verwendung von wenigstens einem substituierten 2-Aminoalkylbiphenyl-Derivat der allgemeinen Formel I in Form seiner Base und/oder eines Salzes einer physiologisch verträglichen Säure zur Herstellung eines Arzneimittels zur Behandlung/Bekämpfung bei/von Schmerzen, inflammatorischen und allergischen Reaktionen, Depressionen, Drogen- und Alkoholmißbrauch, Gastritis, Diarrhoe, Harninkontinenz, cardiovaskulären Erkrankungen, Atemwegserkrankungen, Husten, seelischen Erkrankungen und/oder Epilepsie.

Zur Zubereitung entsprechender pharmazeutischer Formulierungen werden neben mindestens einem substituierten 2-Aminoalkylbiphenyl-Derivat der allgemeinen Formel 1 Trägermaterialien, Füllstoffe, Lösungsmittel, Verdünnungsmittel, Farbstoffe und/oder Bindemittel eingesetzt. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel 1 in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel I verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,5 bis 500 mg/kg wenigstens eines 2-Aminoalkylbiphenyl-Derivates der allgemeinen Formel I appliziert.

Beispiele

Die folgenden Beispiele dienen der Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe Ether bedeutet Diethylether.

Als stationäre Phase für die Säulenchromatographie wurde Kieselgel 60 (0.040 - 0.063 mmm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Vol.% bedeutet Volumenprozent und m% bedeutet Massenprozent.

Beispiel 1:

(3'-Methoxybiphenyl-2-ylmethyl)dimethylamin-Hydrochlorid

1. Stufe

3-Methoxybenzolboronsäure

41,3 g (220 mmol) 3-Bromanisol wurden in 880 ml Tetrahydrofuran gelöst und in einem Kältebad (Ethanol/Trockeneis) auf -70°C gekühlt. 160 ml (250 mmol) Butyllithiumlösung (1,6 M in Hexan) wurden unter Stickstoff so zugetropft, daß die Temperatur nicht über -60 °C stieg. Nach 1,5 Stunden Rühren bei -70°C wurden 75 ml (660 mmol) Trimethylborat ebenfalls so zugetropft, daß die Temperatur nicht über -60 °C stieg. Nach einer weiteren Stunde Rühren in der Kälte wurde innerhalb von zwei Stunden auf 25°C erwärmt, 720 ml Salzsäure (1 M) zugegeben und 15 Stunden bei 25°C gerührt.
Zur Aufarbeitung wurde der Ansatz dreimal mit je 300 ml Ether extrahiert, die organischen Phasen vereinigt, mit je 100 ml Wasser und gesättigter Natriumchloridlösung gewaschen, über wasserfreiem Magnesiumsulfat getrocknet, filtriert und am Rotationsverdampfer (500-10 mbar) eingeengt. Auf diese Weise wurden 30,8 g 3-Methoxybenzolboronsäure (92,1 % der Theorie) erhalten.

2. Stufe

(2-Brombenzyl)dimethylamin

25,1 g (113 mmol) 2-Brombenzylamin-Hydrochlorid wurden in 26 ml (678 mmol) Ameisensäure und 52 ml (678 mmol) Formaldehydlösung (36 m% in Wasser) gelöst und unter Rühren für 6 Stunden auf 95 °C erhitzt. Anschließend wurde die Lösung im Eisbad auf 0 °C gekühlt und 90 g kalte Kaliumhydroxidlösung (50 m%) zugegeben. Es wurde bei 25°C dreimal mit je 100 ml Ether extrahiert, die organischen Phasen vereinigt, mit etwas Aktivkohle versetzt, über wasserfreiem Magnesiumsulfat getrocknet, filtriert und am Rotationsverdampfer (500-10 mbar) eingeengt. Auf diese Weise wurden 22,2 g (2-Brombenzyl)dimethylamin (91,9 % der Theorie) erhalten.

3. Stufe

(3'-Methoxybiphenyl-2-ylmethyl)dimethylamin-Hydrochlorid

1,13 g (7,43 mmol) 3-Methoxybenzolboronsäure, 1,67 g (7,78 mmol) (2-Brombenzyl)dimethylamin und 2,62 g

(24,7 mmol) Natriumcarbonat wurden in einer Mischung aus 50 ml Toluol, 20 ml Wasser und 10 ml Ethanol gelöst. Unter Stickstoff wurden 175 mg Tetrakis(triphenylphosphin)palladium(0) zugegeben und unter Rühren für 16 Stunden auf 110 °C erhitzt.
Zur Aufarbeitung wurden 75 ml Ether zugegeben und dreimal mit je 75 ml einer Kaliumhydroxidlösung (0,5 M) extrahiert. Die vereinigten wässrigen Lösungen wurden mit 30 ml Ether extrahiert, die vereinigten organischen Phasen über wasserfreiem Magnesiumsulfat getrocknet, filtriert und am Rotationsverdampfer (500-10 mbar) eingeengt. Es wurden 2,12 g Rohbase (118 % der Theorie) erhalten, die auf eine 3 x 25 cm Säule, gefüllt mit Kieselgel, gegeben wurden. Die Elution mit Ether/n-Hexan 1:1 (V:V) ergab 0,61 g Base, die in 6,0 ml 2-Butanon gelöst und nacheinander mit 25 µl (1,39 mmol) Wasser und 350 µl (2,78 mmol) Chlortrimethylsilan versetzt wurde. Der Ansatz wurde 15 Stunden bei 25°C aufbewahrt, der ausgefallene Feststoff abfiltriert, mit kleinen Portionen Ether gewaschen und im Ölpumpenvakuum bis zur Gewichtskonstanz getrocknet. Auf diese Weise wurden 0,56 g (27,2 % der Theorie) (3'-Methoxybiphenyl-2-ylmethyl)dimethylamin-Hydrochlorid mit einem Schmelzpunkt von 144 °C erhalten.

Beispiel 2:

(4'-Chlorbiphenyl-2-ylmethyl)dimethylamin-Hydrochlorid

0,88 g (5,65 mmol) 4-Chlorbenzolboronsäure, 1,27 g (5,93 mmol) des nach Beispiel 1 (2. Stufe) hergestellten (2-Brombenzyl)dimethylamin und 2,00 g (18,8 mmol) Natriumcarbonat wurden in einer Mischung aus 39 ml Toluol, 16 ml Wasser und 8 ml Ethanol gelöst. Unter Stickstoff wurden 133 mg Tetrakis(triphenylphosphin)palladium(0) zugegeben und 16 Stunden zum Rückfluß erhitzt (Badtemperatur 110 °C).
Zur Aufarbeitung wurden 65 ml Ether zugegeben und dreimal mit je 65 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500-10 mbar) eingeengt. Es wurden 1,30 g Rohbase (93,8 % der Theorie) erhalten, die auf eine 3 x 25 cm Säule, gefüllt mit Kieselgel, gegeben wurden. Die Elution mit Ether/n-Hexan 1:3 (V:V) ergab 0,61 g Base, die zur weiteren Reinigung per HPLC aufgetrennt wurden. Trennbedingungen: Fließmittel Acetonitril-Wasser (80:20 (V/V) + 0,5 Vol.-% Isopropylamin), Fluß 10 ml/min, Wellenlänge 254 nm, Säule Eurogel PRP 100 (Hersteller Knauer, 250 x 16 mm, mit Vorsäule). Es wurden 0,31 g Rohbase erhalten, aus der nach Beispiel 1 (3. Stufe) mit Chlortrimethylsilan/Wasser in 2-Butanon 0,33 g (20,7 % der Theorie) (4'-Chlorbiphenyl-2-ylmethyl)dimethylamin-Hydrochlorid mit einem Schmelzpunkt von 232 °C erhalten wurden.

Beispiel 3:

2'-Dimethylaminomethylbiphenyl-3-ol-Hydrochlorid

0,70 g (2,52 mmol) des nach Beispiel 1 (3. Stufe) hergestellten (3'-Methoxybiphenyl-2-ylmethyl)dimethylamin-Hydrochlorid wurden in 10 ml Wasser gelöst, die Base mit 10 ml Wasser und 2 ml Natronlauge (32 m%) freigesetzt, dreimal mit je 20 ml Ether extrahiert, die vereinigten organischen Extrakte über wasserfreiem Magnesiumsulfat getrocknet, filtriert und am Rotationsverdampfer (500 bis 10 mbar) eingeengt. 0,59 g (2,44 mmol) dieser Base wurden mit 55 ml Bromwasserstofflösung (48 m% in Wasser) für zwei Stunden zum Rückfluß erhitzt (Badtemperatur 145 °C).
Zur Aufarbeitung wurde der Ansatz in 600 ml Natriumhydrogencarbonatlösung (1 M) gegossen (pH 7-8), dreimal mit je 100 ml Essigsäureethylester extrahiert, die vereinigten organischen Extrakte über wasserfreiem Magnesiumsulfat getrocknet, filtriert und am Rotationsverdampfer (500 bis 10 mbar) eingeengt. Es wurden 0,61 g Rohbase (109 % der Theorie) erhalten, die auf eine 3 x 25 cm Säule, gefüllt mit Kieselgel, gegeben wurden. Die Elution mit Ether ergab 0,51 g Base, aus der nach Beispiel 1 (3. Stufe) mit Chlortrimethylsilan/Wasser in 2-Butanon 0,51 g (79,7 % der Theorie) 2'-Dimethylaminomethylbiphenyl-3-ol-Hydrochlorid mit einem Schmelzpunkt von 180 °C erhalten wurden.

Beispiel 4:

(2'-Methoxybiphenyl-2-ylmethyl)dimethylamin-Hydrochlorid

1,00 g (6,58 mmol) 2-Methoxybenzolboronsäure, 1,48 g (6,91 mmol) des nach Beispiel 1 (2. Stufe) hergestellten (2-Brombenzyl)dimethylamin und 2,32 g (21,9 mmol) Natriumcarbonat wurden in einer Mischung aus 45 ml Toluol, 18 ml Wasser und 9 ml Ethanol gelöst. Unter Stickstoff wurden 160 mg Tetrakis(triphenylphosphin)palladium(0) zugegeben und 16 Stunden zum Rückfluß erhitzt (Badtemperatur 110 °C).
Zur Aufarbeitung wurden 75 ml Ether zugegeben und dreimal mit je 75 ml Kaliumhydroxidlösung (0,5 M) extrahiert. Die vereinigten wässrigen Lösungen wurden mit 20 ml Ether extrahiert, die vereinigten organischen Phasen über wasserfreiem Magnesiumsulfat getrocknet, filtriert und am Rotationsverdampfer (500 bis 10 mbar) eingeengt. Es wurden 1,62

g Rohbase (102 % der Theorie) erhalten, die auf eine 3 x 25 cm Säule, gefüllt mit Kieselgel, gegeben wurden. Die Elution mit Ether/n-Hexan 1:2 (V:V) ergab 0,64 g Base, aus der nach Beispiel 1 (3. Stufe) mit Chlortrimethylsilan/Wasser in 2-Butanon 0,31 g (17,1 % der Theorie) (2'-Methoxybiphenyl-2-ylmethyl)dimethylamin-Hydrochlorid mit einem Schmelzpunkt von 163 °C erhalten wurden.

Beispiel 5:

(3'-Chlorbiphenyl-2-ylmethyl)dimethylamin-Hydrochlorid

1,00 g (6,39 mmol) 3-Chlorbenzolboronsäure, 1,44 g (6,71 mmol) des nach Beispiel 1 (2. Stufe) hergestellten (2-Brombenzyl)dimethylamin und 2,26 g (21,3 mmol) Natriumcarbonat wurden in einer Mischung aus 44 ml Toluol, 17 ml Wasser und 9 ml Ethanol gelöst. Unter Stickstoff wurden 160 mg Tetrakis(triphenylphosphin)palladium(0) zugegeben und 16 Stunden zum Rückfluß erhitzt (Badtemperatur 110 °C).
Zur Aufarbeitung wurden 75 ml Ether zugegeben und dreimal mit je 75 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 bis 10 mbar) eingeengt. Es wurden 1,49 g Rohbase (94,7 % der Theorie) erhalten, die auf eine 3 x 25 cm Säule, gefüllt mit Kieselgel, gegeben wurden. Die Elution mit Ether/n-Hexan 1:1 (V:V) ergab 0,62 g Base, aus der nach Beispiel 1 (3. Stufe) mit Chlortrimethylsilan/Wasser in 2-Butanon ein Hydrochlorid gefällt wurde. Hieraus wurde mit 10 ml Wasser und 2 ml Natriumhydroxidlösung (32 m%) die Base freigesetzt, dreimal mit je 20 ml Ether extrahiert, die vereinigten organischen Extrakte über wasserfreiem Magnesiumsulfat getrocknet, filtriert und am Rotationsverdampfer (500 bis 10 mbar) eingeengt. Die erhaltene Rohbase wurde per HPLC gereinigt. Trennbedingungen: Fließmittel Acetonitril - Wasser (80:20 (V/V) + 0,5 Vol.-% Isopropylamin), Fluß 10 ml/min, Wellenlänge 254 nm, Säule Eurogel PRP 100 (Hersteller Knauer, 250 x 16 mm, mit Vorsäule). Es wurden 0,32 g Rohbase erhalten, aus der nach Beispiel 1 (3. Stufe) mit Chlortrimethylsilan/Wasser in 2-Butanon 0,29 g (16,3 % der Theorie) (3'-Chlorbiphenyl-2-ylmethyl)dimethylamin-Hydrochlorid mit einem Schmelzpunkt von 169 °C erhalten wurden.

Beispiel 6:

(2'-Fluorbiphenyl-2-ylmethyl)dimethylamin-Hydrochlorid

1,02 g (7,27 mmol) 2-Fluorbenzolboronsäure, 1,63 g (7,63 mmol) des nach Beispiel 1 (2. Stufe) hergestellten (2-Brombenzyl)dimethylamin und 2,57 g (24,2 mmol) Natriumcarbonat wurden in einer Mischung aus 50 ml Toluol, 20 ml Wasser und 10 ml Ethanol gelöst. Unter Stickstoff wurden 172 mg Tetrakis(triphenylphosphin)palladium(0) zugegeben und 16 Stunden zum Rückfluß erhitzt (Badtemperatur 110 °C).
Zur Aufarbeitung wurden 80 ml Ether zugegeben und dreimal mit je 80 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 bis 10 mbar) eingeengt. Es wurden 1,73 g Rohbase (104 % der Theorie) erhalten, die auf eine 3 x 25 cm Säule, gefüllt mit Kieselgel, gegeben wurden. Die Elution mit Ether/n-Hexan 1:3 (V:V) ergab 0,13 g Base, aus der nach Beispiel 1 (3. Stufe) mit Chlortrimethylsilan/Wasser in 2-Butanon 0,10 g (4,7 % der Theorie) (2'-Fluorbiphenyl-2-ylmethyl)dimethylamin-Hydrochlorid mit einem Schmelzpunkt von 184 °C erhalten wurden.

Beispiel 7:

(3'-Fluorbiphenyl-2-ylmethyl)dimethylamin-Hydrochlorid

1,03 g (7,39 mmol) 3-Fluorbenzolboronsäure, 1,05 g (4,93 mmol) des nach Beispiel 1 (2. Stufe) hergestellten (2-Brombenzyl)dimethylamin und 2,61 g (24,6 mmol) Natriumcarbonat wurden in einer Mischung aus 50 ml Toluol, 20 ml Wasser und 10 ml Ethanol gelöst. Unter Stickstoff wurden 175 mg Tetrakis(triphenylphosphin)palladium(0) zugegeben und 16 Stunden zum Rückfluß erhitzt (Badtemperatur 110 °C).
Zur Aufarbeitung wurden 80 ml Ether zugegeben und dreimal mit je 80 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,38 g Rohbase (122 % der Theorie) erhalten, die auf eine 3 x 25 cm Säule, gefüllt mit Kieselgel, gegeben wurden. Die Elution mit Ether/n-Hexan 1:3 (V:V) ergab 0,57 g Base, aus der nach Beispiel 1 (3. Stufe) mit Chlortrimethylsilan/Wasser in 2-Butanon 0,53 g (41,9 % der Theorie) (3'-Fluorbiphenyl-2-ylmethyl)dimethylamin-Hydrochlorid mit einem Schmelzpunkt von 183 °C erhalten wurden.

Beispiel 8:

(4'-Fluorbiphenyl-2-ylmethyl)dimethylamin-Hydrochlorid

1,00 g (7,15 mmol) 4-Fluorbenzolboronsäure, 1,02 g (4,76 mmol) des nach Beispiel 1 (2. Stufe) hergestellten (2-Brombenzyl)dimethylamin und 2,52 g (23,8 mmol) Natriumcarbonat wurden in einer Mischung aus 50 ml Toluol, 20 ml Wasser und 10 ml Ethanol gelöst. Unter Stickstoff wurden 170 mg Tetrakis(triphenylphosphin)palladium(0) zugegeben und 16 Stunden zum Rückfluß erhitzt (Badtemperatur 110 °C).
Zur Aufarbeitung wurden 80 ml Ether zugegeben und dreimal mit je 80 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,21 g Rohbase (111 % der Theorie) erhalten, die auf eine 3 x 25 cm Säule, gefüllt mit Kieselgel, gegeben wurden. Die Elution mit Ether/n-Hexan 1:3 (V:V) ergab 0,56 g Base, aus der nach Beispiel 1 (3. Stufe) mit Chlortrimethylsilan/Wasser in 2-Butanon 0,56 g (44,0 % der Theorie) (4'-Fluorbiphenyl-2-ylmethyl)dimethylamin-Hydrochlorid mit einem Schmelzpunkt von 222 °C erhalten wurden.

Beispiel 9:

(3'-Chlor-4'-fluorbiphenyl-2-ylmethyl)dimethylamin-Hydrochlorid

1,12 g (6,41 mmol) 3-Chlor-4-fluorbenzolboronsäure, 1,44 g (6,73 mmol) des nach Beispiel 1 (2. Stufe) hergestellten (2-Brombenzyl)dimethylamin und 2,26 g (21,4 mmol) Natriumcarbonat wurden in einer Mischung aus 44 ml Toluol, 18 ml Wasser und 9 ml Ethanol gelöst. Unter Stickstoff wurden 151 mg Tetrakis(triphenylphosphin)palladium(0) zugegeben und 16 Stunden zum Rückfluß erhitzt (Badtemperatur 110 °C).
Zur Aufarbeitung wurden 70 ml Ether zugegeben und dreimal mit je 70 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,66 g Rohbase (98,2 % der Theorie) erhalten, die auf eine 3 x 25 cm Säule, gefüllt mit Kieselgel, gegeben wurden. Die Elution mit Ether/n-Hexan 1:1 (V:V) ergab 0,66 g Base, die per HPLC gereinigt wurde. Trennbedingungen: Fließmittel Acetonitril - Wasser (80:20 (V/V) + 0,5 Vol.-% Isopropylamin), Fluß 10 ml/min, Wellenlänge 254 nm, Säule Eurogel PRP 100 (Hersteller Knauer, 250 x 4,6 mm, mit Vorsäule). Es wurden 0,37 g Rohbase erhalten, aus der nach Beispiel 1 (3. Stufe) mit Chlortrimethylsilan/Wasser in 2-Butanon 0,34 g (17,6 % der Theorie) (3'-Chlor-4'-fluorbiphenyl-2-ylmethyl) dimethylamin-Hydrochlorid mit einem Schmelzpunkt von 205 °C erhalten wurden.

Beispiel 10:

(3'-Methoxybiphenyl-2-ylethyl)dimethylamin-Hydrochlorid

1. Stufe

2-(2-Brom-phenyl)-ethylamin

10,0 g (51,0 mmol) 2-Bromphenylacetonitril wurden in 80 ml Ether gelöst und zu 5,81 g (153 mol) Lithiumaluminiumhydrid in 230 ml Ether getropft. Es wurde unter Rühren für drei Stunden zum Rückfluß erhitzt und nach Abkühlung unter kräftigem Rühren langsam 80 ml Kaliumhydroxidlösung (10 m%) zugetropft. Nach Rühren über Nacht wurde der Überstand abdekantiert, der Rückstand zweimal mit je 100 ml Ether nachgewaschen, die vereinigten organischen Phasen über wasserfreiem Magnesiumsulfat getrocknet, filtriert und am Rotationsverdampfer (500 - 10 mbar) eingeengt. Auf diese Weise wurden 9,48 g 2-(2-Brom-phenyl)-ethylamin (93 % der Theorie) erhalten.

2. Stufe

[2-(2-Bromphenyl)ethyl]dimethylamin

9,42 g (47,3 mmol) 2-(2-Bromphenyl)ethylamin wurden in 18 ml (473 mmol) Ameisensäure und 36 ml (473 mmol) Formaldehydlösung (36 m% in Wasser) gelöst und unter Rühren bei Rückfluß für 6 Stunden auf 95 °C erhitzt. Anschließend wurde die Lösung im Eisbad auf 0 °C gekühlt und 61 g kalte. Kaliumhydroxidlösung (50 m%) zugegeben. Es wurde bei 25°C dreimal mit je 40 ml Ether extrahiert, die organischen Phasen vereinigt, mit etwas Aktivkohle versetzt, über wasserfreiem Magnesiumsulfat getrocknet, filtriert und am Rotationsverdampfer (500 - 10 mbar) eingeengt. Auf

diese Weise wurden 11,3 g leicht verunreinigtes [2-(2-Bromphenyl)ethyl]dimethylamin (105 % der Theorie) erhalten.

3. Stufe

(3'-Methoxybiphenyl-2-ylethyl)dimethylamin-Hydrochlorid

2,0 g (13,2 mmol) der nach Beispiel 1 (1. Stufe) hergestellten 3-Methoxybenzolboronsäure, 3,15 g (13,8 mmol) [2-(2-Bromphenyl)ethyl]dimethylamin aus Stufe 1 und 4,66 g (43,8 mmol) Natriumcarbonat wurden in einer Mischung aus 90 ml Toluol, 36 ml Wasser und 18 ml Ethanol gelöst. Unter Stickstoff wurden 312 mg Tetrakis(triphenylphosphin) palladium(0) zugegeben und 16 Stunden zum Rückfluß erhitzt (Badtemperatur 110 °C).
Zur Aufarbeitung wurden 150 ml Ether zugegeben und dreimal mit je 150 ml Kaliumhydroxidlösung (0,5 M) extrahiert. Die vereinigten wässrigen Lösungen wurden mit 50 ml Ether reextrahiert, die vereinigten organischen Phasen über wasserfreiem Magnesiumsulfat getrocknet, filtriert und am Rotationsverdampfer (500 - 10 mbar) eingeengt. Es wurden 3,52 g Rohbase (104 % der Theorie) erhalten, die auf eine 4,5 x 33 cm Säule, gefüllt mit Kieselgel, gegeben wurden. Die Elution mit Ether/n-Hexan 1:1 (V:V) ergab 2,24 g Base, die per HPLC gereinigt wurde. Trennbedingungen: Fließmittel Acetonitril - Wasser (70:30 (V/V) + 0,05 Vol.-% Isopropylamin), Fluß 10 ml/min, Wellenlänge 254 nm, Säule Eurogel PRP 100 (Hersteller Knauer, 250 x 16 mm, mit Vorsäule). Es wurden 0,96 g Rohbase erhalten, aus der nach Beispiel 1 (3. Stufe) mit Chlortrimethylsilan/Wasser in 2-Butanon 0,65 g (3'-Methoxybiphenyl-2-ylethyl)dimethylamin-Hydrochlorid (17,3 % der Theorie) mit einem Schmelzpunkt von 143 °C erhalten wurden.

Beispiel 11:

Dimethyl-[2-(2-methylbenzofuran-4-yl)benzyl]amin-Hydrochlorid

1. Stufe

2-(Dimethylaminomethyl)benzolboronsäure

23,3 g (109 mmol) des in Beispiel 1 (2. Stufe) hergestellten (2-Brombenzyl)dimethylamin wurden in 400 ml Tetrahydrofuran gelöst und im Kältebad (Ethanol/Trockeneis) auf -70°C gekühlt. 78 ml (125 mmol) Butyllithiumlösung (1,6 M in Hexan) wurden unter Stickstoff so zugetropft, daß die Temperatur nicht über - 65 °C stieg. Nach 1,5 Stunden Rühren bei -70°C wurden 37 ml Trimethylborat so zugetropft, daß die Temperatur nicht über -60 °C stieg. Nach einer weiteren Stunde Rühren in der Kälte wurde innerhalb von zwei Stunden auf 25°C erwärmt, 350 ml Salzsäure (1 M) zugegeben und 15 Stunden bei 25°C gerührt.
Zur Aufarbeitung wurde der Ansatz mit 10 ml Natriumhydroxidlösung (32 m%) neutralisiert, mit 3,5 g Natriumcarbonat alkalisch gestellt (pH ca. 9), dreimal mit je 150 ml Ether extrahiert, die organischen Phasen vereinigt, über wasserfreiem Magnesiumsulfat getrocknet, filtriert und am Rotationsverdampfer (500 - 10 mbar) eingeengt. Auf diese Weise wurden 9,29 g 2-(Dimethylaminomethyl)benzolboronsäure (47,7 % der Theorie) erhalten.

2. Stufe

Dimethyl-[2-(2-methylbenzofuran-4-yl)benzyl]amin-Hydrochlorid

1,00 g (5,59 mmol) 2-(Dimethylaminomethyl)benzolboronsäure aus Stufe 1, 1,24 g (5,86 mmol) 4-Brom-2-methylbenzofuran und 1,97 g (18,6 mmol) Natriumcarbonat wurden in einer Mischung aus 38 ml Toluol, 15 ml Wasser und 8 ml Ethanol gelöst. Unter Stickstoff wurden 132 mg Tetrakis(triphenylphosphin)palladium(0) zugegeben und 16 Stunden zum Rückfluß erhitzt (Badtemperatur 110°C).
Zur Aufarbeitung wurden 75 ml Ether zugegeben und dreimal mit je 75 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,75 g Rohbase (124 % der Theorie) erhalten, die auf eine 3 x 25 cm Säule, gefüllt mit Kieselgel, gegeben wurden. Die Elution mit Ether/n-Hexan 1:3 (V:V) ergab 0,78 g Base, aus der nach Beispiel 1 (3. Stufe) mit Chlortrimethylsilan/Wasser in 2-Butanon 0,64 g (39,5 % der Theorie) Dimethyl-[2-(2-rriethylbenzofuran-4-yl)benzyl]amin-Hydrochlorid mit einem Schmelzpunkt von 217 °C erhalten wurden.

Beispiel 12:

2'-Dimethylaminomethylbiphenyl-2-carbaldehyd-Hydrochlorid

1,20 g (7,97 mmol) 2-Formylbenzolboronsäure, 1,63 g (7,59 mmol) des nach Beispiel 1 (2. Stufe) hergestellten (2-Brombenzyl)dimethylamin und 2,68 g (25,3 mmol) Natriumcarbonat wurden in einer Mischung aus 52 ml Toluol, 21 ml Wasser und 10 ml Ethanol gelöst. Unter Stickstoff wurden 180 mg Tetrakis(triphenylphosphin)palladium(0) zugegeben und 16 Stunden zum Rückfluß erhitzt (Badtemperatur 110 °C).
Zur Aufarbeitung wurden 85 ml Ether zugegeben und dreimal mit je 85 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,87 g Rohbase (98,2 % der Theorie) erhalten. Die Base wurde in 50 ml Ether gelöst, dreimal mit je 25 ml Salzsäure (5 m%) extrahiert, die vereinigten sauren Phasen mit 10 ml Ether gewaschen und mit 15 ml Natronlauge (32 m%) alkalisch gestellt (pH ca. 11). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,28 g Rohbase (70,3 % der Theorie) erhalten, die auf eine 3 x 25 cm Säule, gefüllt mit Kieselgel, gegeben wurden. Die Elution mit Ether/n-Hexan 1:1 (V:V) ergab 0,42 g Base, aus der nach Beispiel 1 (3. Stufe) mit Chlortrimethylsilan/Wasser in 2-Butanon 0,43 g (20,5 % der Theorie) 2'-Dimethylaminomethylbiphenyl-2-carbaldehyd-Hydrochlorid mit einem Schmelzpunkt von 230 °C erhalten wurden.

Beispiel 13:

(3'-Difluormethylbiphenyl-2-ylmethyl)dimethylamin-Hydrochlorid

0,98 g (5,46 mmol) des nach Beispiel 11 (1. Stufe) hergestellten 2-(Dimethylaminomethyl)benzolboronsäure , 1,19 g (5,73 mmol) 1-Brom-3-difluormethylbenzol und 1,93 g (18,2 mmol) Natriumcarbonat wurden in einer Mischung aus 37 ml Toluol, 15 ml Wasser und 8 ml Ethanol gelöst. Unter Stickstoff wurden 130 mg Tetrakis(triphenylphosphin) palladium(0) zugegeben und 16 Stunden zum Rückfluß erhitzt (Badtemperatur 110 °C).
Zur Aufarbeitung wurden 60 ml Ether zugegeben und dreimal mit je 60 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,46 g Rohbase (103 % der Theorie) erhalten, die auf eine 3 x 25 cm Säule, gefüllt mit Kieselgel, gegeben wurden. Die Elution mit Ether/n-Hexan 1:3 (V:V) ergab 0,79 g Base, aus der nach Beispiel 1 (3. Stufe) mit Chlortrimethylsilan/Wasser in 2-Butanon 0,67 g (40,9 % der Theorie) (3'-Difluormethylbiphenyl-2-ylmethyl)dimethylamin-Hydrochlorid mit einem Schmelzpunkt von 147 °C erhalten wurden.

Beispiel 14:

2'-Dimethylaminomethylbiphenyl-3-carbaldehyd-Hydrochlorid

1,03 g (6,89 mmol) 3-Formylbenzolboronsäure, 1,40 g (6,56 mmol) des nach Beispiel 1 (2. Stufe) hergestellten (2-Brombenzyl)dimethylamin und 2,32 g (21,8 mmol) Natriumcarbonat wurden in einer Mischung aus 45 ml Toluol, 18 ml Wasser und 9 ml Ethanol gelöst. Unter Stickstoff wurden 156 mg Tetrakis(triphenylphosphin)palladium(0) zugegeben und 16 Stunden zum Rückfluß erhitzt (Badtemperatur 110 °C).
Zur Aufarbeitung wurden 75 ml Ether zugegeben und dreimal mit je 75 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,66 g Rohbase (100 % der Theorie) erhalten. Die Base wurde in 50 ml Ether gelöst, dreimal mit je 25 ml Salzsäure (5 m%) extrahiert, die vereinigten sauren Phasen mit 10 ml Ether gewaschen und mit 15 ml Natronlauge (32 m%) alkalisch gestellt (pH ca. 11). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,08 g Rohbase (68,7 % der Theorie) erhalten, die auf eine 3 x 25 cm Säule, gefüllt mit Kieselgel, gegeben wurden. Die Elution mit Ether/n-Hexan 1:3 (V:V) ergab 0,40 g Base, aus der nach Beispiel 1 (3. Stufe) mit Chlortrimethylsilan/Wasser in 2-Butanon 0,31 g (17,3 % der Theorie) 2'-Dimethylaminomethylbiphenyl-3-carbaldehyd-Hydrochlorid mit einem Schmelzpunkt von 185 °C erhalten wurden.

Beispiel 15:

Biphenyl-2-ylmethyldimethylamin-Hydrochlorid

1,01 g (8,30 mmol) Benzolboronsäure, 1,69 g (7,90 mmol) des nach Beispiel 1 (2. Stufe) hergestellten (2-Brombenzyl)dimethylamin und 2,79 g (26,3 mmol) Natriumcarbonat wurden in einer Mischung aus 54 ml Toluol, 22 ml Wasser und 11 ml Ethanol gelöst. Unter Stickstoff wurden 187 mg Tetrakis(triphenylphosphin)palladium(0) zugegeben und 16 Stunden zum Rückfluß erhitzt (Badtemperatur 110 °C).
Zur Aufarbeitung wurden 90 ml Ether zugegeben und dreimal mit je 90 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,64 g Rohbase (93,3 % der Theorie) erhalten, die auf eine 3 x 25 cm Säule, gefüllt mit Kieselgel, gegeben wurden. Die Elution mit Ether/n-Hexan 1:3 (V:V) ergab 0,26 g Base, aus der nach Beispiel 1 (3. Stufe) mit Chlortrimethylsilan/Wasser in 2-Butanon 0,28 g (14,2 % der Theorie) Biphenyl-2-ylmethyldimethylamin-Hydrochlorid mit einem Schmelzpunkt von 189 °C erhalten wurden.

Beispiel 16:

(3',4'-Dichlorbiphenyl-2-ylmethyl)dimethylamin-Hydrochlorid

1,01 g (5,29 mmol) 3,4-Dichlorbenzolboronsäure, 1,19 g (5,56 mmol) des nach Beispiel 1 (2. Stufe) hergestellten (2-Brombenzyl)dimethylamin und 1,87 g (17,6 mmol) Natriumcarbonat wurden in einer Mischung aus 36 ml Toluol, 15 ml Wasser und 7 ml Ethanol gelöst. Unter Stickstoff wurden 125 mg Tetrakis(triphenylphosphin)palladium(0) zugegeben und 16 Stunden zum Rückfluß erhitzt (Badtemperatur 110 °C).
Zur Aufarbeitung wurden 60 ml Ether zugegeben und dreimal mit je 60 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,43 g Rohbase (96,5 % der Theorie) erhalten, die auf eine 3 x 25 cm Säule, gefüllt mit Kieselgel, gegeben wurden. Die Elution mit Ether/n-Hexan 1:3 (V:V) ergab 0,52 g Base, die zur weiteren Reinigung per HPLC aufgetrennt wurde. Trennbedingungen: Fließmittel Acetonitril-Wasser (90:10 (V/V) + 0,05 Vol.-% Isopropylamin), Fluß 10 ml/min, Wellenlänge 254 nm, Säule Eurogel PRP 100 (Hersteller Knauer, 250 x 16 mm, mit Vorsäule). Es wurden 0,20 g Rohbase erhalten, aus der nach Beispiel 1 (3. Stufe) mit Chlortrimethylsilan/Wasser in 2-Butanon 0,19 g (11,4 % der Theorie) (3',4'-Dichlorbiphenyl-2-ylmethyl)dimethylamin-Hydrochlorid mit einem Schmelzpunkt von 219 °C erhalten wurden.

Beispiel 17:

(3',5'-Dichlorbiphenyl-2-ylmethyl)dimethylamin-Hydrochlorid

0,89 g (4,66 mmol) 3,5-Dichlorbenzolboronsäure, 0,95 g (4,44 mmol) des nach Beispiel 1 (2. Stufe) hergestellten (2-Brombenzyl)dimethylamin und 1,57 g (14,8 mmol) Natriumcarbonat wurden in einer Mischung aus 30 ml Toluol, 12 ml Wasser und 6 ml Ethanol gelöst. Unter Stickstoff wurden 106 mg Tetrakis(triphenylphosphin)palladium(0) zugegeben und 16 Stunden zum Rückfluß erhitzt (Badtemperatur 110 °C).
Zur Aufarbeitung wurden 50 ml Ether zugegeben und dreimal mit je 50 ml Kaliumhydroxidlösung (0,5 M) extrahiert. Die vereinigten wässrigen Lösungen wurden mit 20 ml Ether reextrahiert, die vereinigten organischen Phasen über wasserfreiem Magnesiumsulfat getrocknet, filtriert und am Rotationsverdampfer (500 - 10 mbar) eingeengt. Es wurden 1,25 g Rohbase (95,5 % der Theorie) erhalten. Die Base wurde in 50 ml Ether gelöst, dreimal mit je 25 ml Salzsäure (5 m%) extrahiert, die vereinigten sauren Phasen mit 10 ml Ether gewaschen und mit 15 ml Natronlauge (32 m%) alkalisch gestellt (pH ca. 11). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 0,46 g Rohbase (37,3 % der Theorie) erhalten, die auf eine 3 x 15 cm Säule, gefüllt mit Kieselgel, gegeben wurden. Die Elution mit Ether/n-Hexan 1:3 (V:V) ergab 0,23 g Base, aus der nach Beispiel 1 (3. Stufe) mit Chlortrimethylsilan/Wasser in 2-Butanon 0,20 g (14,9 % der Theorie) (3',5'-Dichlorbiphenyl-2-ylmethyl)dimethylamin-Hydrochlorid mit einem Schmelzpunkt von 198 °C erhalten wurden.

Beispiel 18:

Dimethyl-(4'-nitro-3'-trifluormethylbiphenyl-2-ylmethyl)amin-Hydrochlorid

1. Stufe

Dimethyl-2-(dimethylaminomethyl)benzolboronat

20,2 g (94,2 mmol) des nach Beispiel 1 (2. Stufe) hergestellten (2-Brombenzyl)dimethylamin wurden in 350 ml Tetrahydrofuran gelöst und im Kältebad (Isopropanol/Trockeneis) auf -70°C gekühlt. 68 ml (108 mmol) Butyllithiumlösung (1,6 M in Hexan) wurden unter Stickstoff so zugetropft, daß die Temperatur nicht über -60 °C stieg. Nach zwei Stunden Rühren bei -70°C wurden 32 ml (282 mmol) Trimethylborat ebenfalls so zugetropft, daß die Temperatur nicht über -60 °C stieg. Es wurde innerhalb von 15 Stunden auf 25°C erwärmt und die Lösung am Rotationsverdampfer (500 - 10 mbar) ohne Wärmezufuhr eingeengt. Der Rückstand wurde in 200 ml n-Hexan aufgenommen, eine Stunde gerührt, über eine Schutzgasfritte unter Stickstoff abfiltriert und das Filtrat am Rotationsverdampfer (500 - 10 mbar) ohne Wärmezufuhr eingeengt. Auf diese Weise wurden 12,0 g Dimethyl-2-(dimethylaminomethyl)benzolboronat (61,5 % der Theorie) erhalten.

2. Stufe

Dimethyl-(4'-nitro-3'-trifluormethylbiphenyl-2-ylmethyl)amin-Hydrochlorid

1,84 g (8,89 mmol) des nach Stufe 1 hergestellten Dimethyl-2-(dimethylaminomethyl)benzolboronat , 1,20 g (4,44 mmol) 5-Brom-2-nitrobenzotrifluorid und 1,57 g (14,8 mmol) Natriumcarbonat wurden in einer Mischung aus 30 ml Toluol, 12 ml Wasser und 6 ml Ethanol gelöst. Unter Stickstoff wurden 105 mg Tetrakis(triphenylphosphin)palladium (0) zugegeben und 16 Stunden zum Rückfluß erhitzt (Badtemperatur 110 °C).
Zur Aufarbeitung wurden 45 ml Ether zugegeben und dreimal mit je 45 ml Kaliumhydroxidlösung (0,5 M) gewaschen. Die organische Lösung wurde dreimal mit je 17 ml Salzsäure (5 m%) extrahiert, die vereinigten sauren Phasen mit 10 ml Ether gewaschen und mit 10 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,29 g Rohbase (89,4 % der Theorie) erhalten, die auf eine 3 x 15 cm Säule, gefüllt mit Kieselgel, gegeben wurden. Die Elution mit Ether/n-Hexan 1:10 (V:V) ergab 1,05 g Base, aus der nach Beispiel 1 (3. Stufe) mit Chlortrimethylsilan/Wasser in 2-Butanon 1,02 g (66,1 % der Theorie) Dimethyl-(4'-nitro-3'-trifluormethylbiphenyl-2-ylmethyl)amin-Hydrochlorid mit einem Schmelzpunkt oberhalb von 240 °C erhalten wurden.

Beispiel 19:

(3',4'-Difluorbiphenyl-2-ylmethyl)dimethylamin-Hydrochlorid

1,01 g (4,87 mmol) des nach Beispiel 18 (1. Stufe) hergestellten Dimethyl-2-(dimethylaminomethyl)benzolboronat, 1,88 g (9,75 mmol) 3,4-Difluorbrombenzol und 1,72 g (16,2 mmol) Natriumcarbonat wurden in einer Mischung aus 33 ml Toluol, 13 ml Wasser und 7 ml Ethanol gelöst. Unter Stickstoff wurden 116 mg Tetrakis(triphenylphosphin) palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen. Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 0,67 g Rohbase (55,3 % der Theorie) erhalten, die auf eine 3 x 15 cm Säule, gefüllt mit Kieselgel, gegeben wurden. Die Elution mit Ether/n-Hexan 1:10 (V:V) ergab 0,47 g Base, aus der nach Beispiel 1 (3. Stufe) mit Chlortrimethylsilan/Wasser in 2-Butanon 0,52 g (37,6 % der Theorie) (3',4'-Difluorbiphenyl-2-ylmethyl)dimethylamin-Hydrochlorid mit einem Schmelzpunkt von 222 °C erhalten wurden.

Beispiel 20:

(4'-Fluor-3'-trifluormethylbiphenyl-2-ylmethyl)dimethyl-amin-Hydrochlorid

1,02 g (4,91 mmol) des nach Beispiel 18 (1. Stufe) hergestellten Dimethyl-2-(dimethylaminomethyl)benzolboronat , 2,38 g (9,81 mmol) 5-Brom-2-fluorbenzotrifluorid und 1,73 g (16,3 mmol) Natriumcarbonat wurden in einer Mischung aus 34 ml Toluol, 14 ml Wasser und 7 ml Ethanol gelöst. Unter Stickstoff wurden 117 mg Tetrakis (triphenylphosphin)palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen. Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 0,55 g Rohbase (37,5 % der Theorie) erhalten, die auf eine 3 x 15 cm Säule, gefüllt mit Kieselgel, gegeben wurden. Die Elution mit Ether/n-Hexan 1:10 (V:V) ergab 0,39 g Base, aus der nach Beispiel 1 (3. Stufe) mit Chlortrimethylsilan/Wasser in 2-Butanon 0,37 g (22,8 % der Theorie) (4'-Fluor-3'-trifluormethylbiphenyl-2-ylmethyl)dimethyl-amin-Hydrochlorid mit einem Schmelzpunkt von 180 °C erhalten wurden.

Beispiel 21:

(4'-Chlor-3'-methoxybiphenyl-2-ylmethyl)dimethylamin-Hydrochlorid

1,52 g (4,87 mmol) des nach Beispiel 18 (1. Stufe) hergestellten Dimethyl-2-(dimethylaminomethyl) benzolboronat , 1,08 g (4,88 mmol) 5-Brom-2-chlormethoxybenzol und 1,72 g (16,3 mmol) Natriumcarbonat wurden in einer Mischung aus 33 ml Toluol, 13 ml Wasser und 7 ml Ethanol gelöst. Unter Stickstoff wurden 116 mg Tetrakis (triphenylphosphin)palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen. Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,26 g Rohbase (93,3 % der Theorie) erhalten, die auf eine 3 x 15 cm Säule, gefüllt mit Kieselgel, gegeben wurden. Die Elution mit Ether/n-Hexan 1:20 (V:V) ergab 0,44 g Base, aus der nach Beispiel 1 (3. Stufe) mit Chlortrimethylsilan/Wasser in 2-Butanon 0,46 g (29,9 % der Theorie) (4'-Chlor-3'-methoxybiphenyl-2-ylmethyl)dimethylamin-Hydrochlorid mit einem Schmelzpunkt von 218 °C erhalten wurden.

Beispiel 22:

*N*-(2'-Dimethylaminomethyl-3-trifluormethoxybiphenyl-4-yl)acetamid-Hydrochlorid

1,55 g (7,48 mmol) des nach Beispiel 18 (1. Stufe) hergestellten Dimethyl-2-(dimethylaminomethyl)benzolboronat, 1,49 g (4,99 mmol) 4-Brom-2-(trifluormethoxy)acetanilid und 1,76 g (16,6 mmol) Natriumcarbonat wurden in einer Mischung aus 34 ml Toluol, 14 ml Wasser und 7 ml Ethanol gelöst. Unter Stickstoff wurden 118 mg Tetrakis(triphenylphosphin)palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen. Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,72 g Rohbase (97,6 % der Theorie) erhalten, die auf eine 3 x 15 cm Säule, gefüllt mit Kieselgel, gegeben wurden. Die Elution mit Ether/n-Hexan 1:10 (V:V) ergab 0,91 g Base. Aus 0,40 g dieser Base wurden nach Beispiel 1 (3. Stufe) mit Chlortrimethylsilan/Wasser in 2-Butanon 0,39 g (45,5 % der Theorie) N-(2'-Dimethylaminomethyl-3-trifluormethoxybiphenyl-4-yl)acetamid-Hydrochlorid mit einem Schmelzpunkt von 182 °C erhalten.

Beispiel 23:

(3'-Isopropoxybiphenyl-2-ylmethyl)dimethylamin-Hydrochlorid

1,51 g (7,31 mmol) des nach Beispiel 18 (1. Stufe) hergestellten Dimethyl-2-(dimethylaminomethyl) benzolboronat , 1,05 g (4,87 mmol) 1-Brom-3-isopropoxybenzol und 1,72 g (16,2 mmol) Natriumcarbonat wurden in einer Mischung aus 33 ml Toluol, 13 ml Wasser und 7 ml Ethanol gelöst. Unter Stickstoff wurden 116 mg Tetrakis (triphenylphosphin)palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen. Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml

Ether extrahiert, die vereinigten organischen Phasen über wasserfreiem Magnesiumsulfat getrocknet, filtriert und am Rotationsverdampfer (500 - 10 mbar) eingeengt. Es wurden 1,25 g Rohbase (94,8 % der Theorie) erhalten, die auf eine 3 x 15 cm Säule, gefüllt mit Kieselgel, gegeben wurden. Die Elution mit Ether/n-Hexan 1:20 (V:V) ergab 0,65 g Base, aus der nach Beispiel 1 (3. Stufe) mit Chlortrimethylsilan/Wasser in 2-Butanon 0,36 g (24,0 % der Theorie) N-(2'-Dimethylaminomethyl-3-trifluormethoxybiphenyl-4-yl)acetamid-Hydrochlorid erhalten wurden.

Beispiel 24:

2'-(2-Dimethylaminoethyl)biphenyl-3-ol-Hydrochlorid

0,89 g (3,49 mmol) der nach Beispiel 10 (2. Stufe) hergestellten Base von (3'-Methoxybiphenyl-2-ylethyl) dimethylamin-Hydrochlorid (10) wurden mit 89 ml Bromwasserstofflösung (48 m% in Wasser) für zwei Stunden zum Rückfluß erhitzt (Badtemperatur 145°C).
Zur Aufarbeitung wurde der Ansatz in 1000 ml Natriumhydrogencarbonatlösung (1 M) gegossen (pH 7-8), viermal mit je 100 ml Ether extrahiert, die vereinigten organischen Extrakte über wasserfreiem Magnesiumsulfat getrocknet, filtriert und am Rotationsverdampfer (500 bis 10 mbar) eingeengt. Es wurden 0,32 g Rohbase (38,5 % der Theorie) erhalten, aus der nach Beispiel 1 (3. Stufe) mit Chlortrimethylsilan/Wasser in 2-Butanon 0,26 g (27,9 % der Theorie) 2'-(2-Dimethylaminoethyl)biphenyl-3-ol-Hydrochlorid mit einem Schmelzpunkt von 161 °C erhalten wurden.

Beispiel 25:

4-Chlor-2'-dimethylaminomethylbiphenyl-3-ol-Hydrochlorid

0,59 g (2,15 mmol) der nach Beispiel 21 hergestellten Base von (4'-Chlor-3'-methoxybiphenyl-2-ylmethyl) dimethylamin, Hydrochlorid (21) wurden mit 60 ml Bromwasserstofflösung (48 m% in Wasser) für zwei Stunden zum Rückfluß erhitzt (Badtemperatur 145 °C).
Zur Aufarbeitung wurde der Ansatz in 140 ml Wasser gegossen und durch Zugabe von festem Natriumhydrogencarbonat auf einen pH-Wert von 7 - 8 gebracht, dreimal mit je 50 ml Ether extrahiert, die vereinigten organischen Extrakte über wasserfreiem Magnesiumsulfat getrocknet, filtriert und am Rotationsverdampfer (500 bis 10 mbar) eingeengt. Es wurden 0,55 g Rohbase (98,1 % der Theorie) erhalten, aus der nach Beispiel 1 (3. Stufe) mit Chlortrimethylsilan/Wasser in 2-Butanon 0,56 g (88,0 % der Theorie) 4-Chlor-2'-dimethylaminomethylbiphenyl-3-ol-Hydrochlorid mit einem Schmelzpunkt von 194 °C erhalten wurden.

Beispiel 26:

[2-(1*H*-Indol-5-yl)benzyl]dimethylamin

4,77 g (23,0 mmol) des nach Beispiel 18 (1. Stufe) hergestellten Dimethyl-2-(dimethylaminomethyl) benzolboronat , 3,01 g (15,4 mmol) 5-Bromindol und 5,42 g (51,1 mmol) Natriumcarbonat wurden in einer Mischung aus 105 ml Toluol, 42 ml Wasser und 21 ml Ethanol gelöst. Unter Stickstoff wurden 364 mg Tetrakis(triphenylphosphin) palladium(0) zugegeben und 16 Stunden zum Rückfluß erhitzt (Badtemperatur 110 °C).
Zur Aufarbeitung wurden 100 ml Ether zugegeben und dreimal mit je 100 ml Kaliumhydroxidlösung (0,5 M) gewaschen. Die organische Lösung wurde dreimal mit je 45 ml Salzsäure (5 m%) extrahiert, die vereinigten sauren Phasen mit 20 ml Ether gewaschen und mit 25 ml Natronlauge (32 m%) alkalisch gestellt (pH ca. 12). Es wurde dreimal mit je 45 ml Ether extrahiert, die vereinigten organischen Phasen über wasserfreiem Magnesiumsulfat getrocknet, filtriert und am Rotationsverdampfer (500 - 10 mbar) eingeengt. Es wurden 2,23 g Rohbase (58,1 % der Theorie) erhalten, die auf eine 3 x 25 cm Säule, gefüllt mit Kieselgel, gegeben wurden. Die Elution mit Ether/n-Hexan 1:1 (V:V) ergab 0,33 g (37,6 % der Theorie) [2-(1*H*-Indol-5-yl)benzyl]dimethylamin (37,6 % der Theorie).

Beispiel 27:

(4'-Methansulfonylbiphenyl-2-ylmethyl)dimethylamin-Hydrochlorid

1,59 g (7,69 mmol) des nach Beispiel 18 (1. Stufe) hergestellten Dimethyl-2-(dimethylaminomethyl) benzolboronat , 1,21 g (5,13 mmol) 4-Bromphenylmethylsulfon und 1,81 g (17,1 mmol) Natriumcarbonat wurden in einer Mischung aus 35 ml Toluol, 14 ml Wasser und 7 ml Ethanol gelöst. Unter Stickstoff wurden 122 mg Tetrakis(triphenylphosphin)palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen.

Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 0,67 g Rohbase (55,3 % der Theorie) erhalten, die auf eine 3 x 15 cm Säule, gefüllt mit Kieselgel, gegeben wurden. Die Elution mit Ether/n-Hexan 1:1 (V:V) ergab 0,65 g Base, aus der nach Beispiel 1 (3. Stufe) mit Chlortrimethylsilan/Wasser in 2-Butanon 0,62 g (37,2 % der Theorie) (4'-Methansulfonylbiphenyl-2-ylmethyl)dimethylamin-Hydrochlorid mit einem Schmelzpunkt von 173 °C erhalten wurden.

Beispiel 28:

(2',4'-Dichlorbiphenyl-2-ylmethyl)dimethylamin-Hydrochlorid

1,69 g (8,17 mmol) des nach Beispiel 18 (1. Stufe) hergestellten Dimethyl-2-(dimethylaminomethyl)benzolboronat, 1,23 g (5,44 mmol) 2,4-Dichlorbenzolbromid und 1,92 g (18,1 mmol) Natriumcarbonat wurden in einer Mischung aus 37 ml Toluol, 15 ml Wasser und 8 ml Ethanol gelöst. Unter Stickstoff wurden 129 mg Tetrakis(triphenylphosphin) palladium(0) zugegeben und 16 Stunden zum Rückfluß erhitzt (Badtemperatur 110 °C).
Zur Aufarbeitung wurden 60 ml Ether zugegeben und dreimal mit je 60 ml Kaliumhydroxidlösung (0,5 M) gewaschen. Die organische Lösung wurde dreimal mit je 24 ml Salzsäure (5 m%) extrahiert, die vereinigten sauren Phasen mit 10 ml Ether gewaschen und mit 14 ml Natronlauge (32 m%) alkalisch gestellt (pH ca. 12). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 0,62 g Rohbase (40,3 % der Theorie) erhalten, die auf eine 3 x 15 cm Säule, gefüllt mit Kieselgel, gegeben wurden. Die Elution mit Ether/n-Hexan 1:3 (V:V) ergab 0,39 g Base, aus der nach Beispiel 1 (3. Stufe) mit Chlortrimethylsilan/Wasser in 2-Butanon 0,25 g (15,2 % der Theorie) (2',4'-Dichlorbiphenyl-2-ylmethyl)dimethylamin-Hydrochlorid mit einem Schmelzpunkt von 170-171 °C erhalten wurden.

Beispiel 29:

(2',3'-Difluorbiphenyl-2-ylmethyl)dimethylamin-Hydrochlorid

1,97 g (9,53 mmol) des nach Beispiel 18 (1. Stufe) hergestellten Dimethyl-2-(dimethylaminomethyl)benzolboronat, 1,23 g (6,35 mmol) 2,3-Difluorbenzolbromid und 2,24 g (21,2 mmol) Natriumcarbonat wurden in einer Mischung aus 43 ml Toluol, 17 ml Wasser und 9 ml Ethanol gelöst. Unter Stickstoff wurden 151 mg Tetrakis(triphenylphosphin) palladium(0) zugegeben und 16 Stunden zum Rückfluß erhitzt (Badtemperatur 110 °C).
Zur Aufarbeitung wurden 70 ml Ether zugegeben und dreimal mit je 70 ml Kaliumhydroxidlösung (0,5 M) gewaschen. Die organische Lösung wurde dreimal mit je 27 ml Salzsäure (5 m%) extrahiert, die vereinigten sauren Phasen mit 10 ml Ether gewaschen und mit 16 ml Natronlauge (32 m%) alkalisch gestellt (pH ca. 12). Es wurde dreimal mit je 30 ml Ether extrahiert, die vereinigten organischen Phasen über wasserfreiem Magnesiumsulfat getrocknet, filtriert und am Rotationsverdampfer (500 - 10 mbar) eingeengt. Es wurden 0,99 g Rohbase (63,2 % der Theorie) erhalten, die auf eine 3 x 15 cm Säule, gefüllt mit Kieselgel, gegeben wurden. Die Elution mit Ether/n-Hexan 1:3 (V:V) ergab 0,61 g Base, aus der nach Beispiel 1 (3. Stufe) mit Chlortrimethylsilan/Wasser in 2-Butanon 0,55 g (34,3 % der Theorie) (2',3'-Difluorbiphenyl-2-ylmethyl)dimethylamin-Hydrochlorid mit einem Schmelzpunkt von 214 °C erhalten wurden.

Beispiel 30:

(2',5'-Difluorbiphenyl-2-ylmethyl)dimethylamin-Hydrochlorid

1,86 g (8,98 mmol) des nach Beispiel 18 (1. Stufe) hergestellten Dimethyl-2-(dimethylaminomethyl)benzolboronat, 1,16 g (5,99 mmol) 2,5-Difluorbenzolbromid und 2,11 g (19,9 mmol) Natriumcarbonat wurden in einer Mischung aus 41 ml Toluol, 17 ml Wasser und 8 ml Ethanol gelöst. Unter Stickstoff wurden 142 mg Tetrakis(triphenylphosphin) palladium(0) zugegeben und 16 Stunden zum Rückfluß erhitzt (Badtemperatur 110 °C).
Zur Aufarbeitung wurden 65 ml Ether zugegeben und dreimal mit je 65 ml Kaliumhydroxidlösung (0,5 M) gewaschen. Die organische Lösung wurde dreimal mit je 26 ml Salzsäure (5 m%) extrahiert, die vereinigten sauren Phasen mit 10 ml Ether gewaschen und mit 15 ml Natronlauge (32 m%) alkalisch gestellt (pH ca. 12). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 0,66 g Rohbase (44,4 % der Theorie) erhalten, die auf eine 3 x 15 cm Säule, gefüllt mit Kieselgel, gegeben wurden. Die Elution mit Ether/n-Hexan 1:3 (V:V) ergab 0,40 g Base, aus der nach Beispiel 1 (3. Stufe) mit Chlortrimethylsilan/Wasser in 2-Butanon 0,36 g (23,4 % der Theorie) (2',5'-Difluorbiphenyl-2-ylmethyl)dimethylamin-Hydrochlorid mit einem Schmelzpunkt von 165 °C erhalten wurden.

Beispiel **31:**

(2-Benzo[1,3]dioxol-5-ylbenzyl)dimethylamin-Hydrochlorid

1,71 g (8,24 mmol) des nach Beispiel 18 (1. Stufe) hergestellten Dimethyl-2-(dimethylaminomethyl) benzolboronat , 1,10 g (5,49 mmol) 4-Brom-1,2-(methylendioxy)benzol und 1,94 g (18,3 mmol) Natriumcarbonat wurden in einer Mischung aus 38 ml Toluol, 15 ml Wasser und 8 ml Ethanol gelöst.
Unter Stickstoff wurden 130 mg Tetrakis(triphenylphosphin)palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen. Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,46 g Rohbase (104 % der Theorie) erhalten, die auf eine 3 x 15 cm Säule, gefüllt mit Kieselgel, gegeben wurden. Die Elution mit Ether/n-Hexan 1:1 (V:V) ergab 1,17 g Base, aus der nach Beispiel 1 (3. Stufe) mit Chlortrimethylsilan/Wasser in 2-Butanon 1,20 g (74,8 % der Theorie) (2-Benzo[1,3] dioxol-5-ylbenzyl)dimethylamin-Hydrochlorid mit einem Schmelzpunkt von 181 °C erhalten wurden.

Beispiel 32:

1-[2'-(2-Dimethylaminoethyl)biphenyl-3-yl)ethanon-Hydrochlorid

2,13 g (13,0 mmol) 3-Acetylbenzolboronsäure, 1,98 g (8,68 mmol) des nach Beispiel 10 hergestellten [2-(2-Bromphenyl)ethyl]dimethylamin und 3,06 g (28,9 mmol) Natriumcarbonat wurden in einer Mischung aus 60 ml Toluol, 23 ml Wasser und 12 ml Ethanol gelöst. Unter Stickstoff wurden 206 mg Tetrakis(triphenylphosphin)palladium(0) zugegeben und 16 Stunden zum Rückfluß erhitzt (Badtemperatur 110 °C).
Zur Aufarbeitung wurden 90 ml Ether zugegeben und dreimal mit je 90 ml Kaliumhydroxidlösung (0,5 M) gewaschen. Die organische Lösung wurde dreimal mit je 35 ml Salzsäure (5 m%) extrahiert, die vereinigten sauren Phasen mit 20 ml Ether gewaschen und mit 20 ml Natronlauge (32 m%) alkalisch gestellt (pH ca. 12). Es wurde dreimal mit je 40 ml Ether extrahiert, die vereinigten organischen Phasen über wasserfreiem Magnesiumsulfat getrocknet, filtriert und am Rotationsverdampfer (500 - 10 mbar) eingeengt. Es wurden 2,08 g Rohbase (89,5 % der Theorie) erhalten, aus der nach Beispiel 1 (3. Stufe) mit Chlortrimethylsilan/Wasser in 2-Butanon 1,57 g (59,6 % der Theorie) 1-[2'-(2-Dimethylaminoethyl)biphenyl-3-yl]ethanon-Hydrochlorid mit einem Schmelzpunkt von 141 °C erhalten wurden.

Beispiel 33:

[2-(3',4'-Dimethoxybiphenyl-2-ylethyl]dimethylamin-Hydrochlorid

2,22 g (12,2 mmol) 3,4-Dimethoxybenzolboronsäure, 1,86 g (8,14 mmol) des nach Beispiel 10 hergestellten [2-(2-Bromphenyl)ethyl]dimethylamin und 2,87 g (27,1 mmol) Natriumcarbonat wurden in einer Mischung aus 55 ml Toluol, 22 ml Wasser und 11 ml Ethanol gelöst. Unter Stickstoff wurden 193 mg Tetrakis(triphenylphosphin)palladium (0) zugegeben und 16 Stunden zum Rückfluß erhitzt (Badtemperatur 110 °C).
Zur Aufarbeitung wurden 90 ml Ether zugegeben und dreimal mit je 90 ml Kaliumhydroxidlösung (0,5 M) gewaschen. Die organische Lösung wurde dreimal mit je 35 ml Salzsäure (5 m%) extrahiert, die vereinigten sauren Phasen mit 20 ml Ether gewaschen und mit 20 ml Natronlauge (32 m%) alkalisch gestellt (pH ca. 12). Es wurde dreimal mit je 35 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,82 g Rohbase (78,0 % der Theorie) erhalten, aus der nach Beispiel 1 (3. Stufe) mit Chlortrimethylsilan/Wasser in 2-Butanon 2,02 g (76,8 % der Theorie) [2-(3',4'-Dimethoxybiphenyl-2-ylethyl]dimethylamin-Hydrochlorid mit einem Schmelzpunkt von 179 °C erhalten wurden.

Beispiel 34:

[2-(3'-Isopropoxybiphenyl-2-yl)ethyl]dimethylamin-Hydrochlorid

1. Stufe

Dimethyl-2-(2-dimethylaminoethyl)benzolboronat

19,0 g (83,2 mmol) des nach Beispiel 10 (2. Stufe) hergestellten [2-(2-Bromphenyl)ethyl]dimethylamin wurden in 300 ml Tetrahydrofuran gelöst und im Kältebad (Isopropanol/Trockeneis) auf -70°C gekühlt. 60 ml (95,7 mmol) Butyllithiumlösung (1,6 M in Hexan) wurden unter Stickstoff so zugetropft, daß die Temperatur nicht über -60 °C stieg. Nach zwei Stunden Rühren bei -70°C wurden 28 ml (250 mmol) Trimethylborat ebenfalls so zugetropft, daß die Temperatur nicht über -60 °C stieg. Es wurde innerhalb von 15 Stunden auf 25°C erwärmt und die Lösung am Rotationsverdampfer (500 - 10 mbar) ohne Wärmezufuhr eingeengt. Der Rückstand wurde in 200 ml n-Hexan aufgenommen, eine Stunde gerührt, über eine Schutzgasfritte unter Stickstoff abfiltriert und das Filtrat am Rotationsverdampfer (500 - 10 mbar) ohne Wärmezufuhr eingeengt. Auf diese Weise wurden 14,1 g Dimethyl-2-(2-dimethylaminoethyl)benzolboronat (76,5 % der Theorie) erhalten.

2. Stufe

[2-(3'-Isopropoxybiphenyl-2-yl)ethyl]dimethylamin-Hydrochlorid

1,20 g (5,43 mmol) Dimethyl-2-(2-dimethylaminoethyl)benzolboronat aus Stufe 1, 1,75 g (8,14 mmol) 3-Bromisopropoxybenzol und 1,92 g (18,1 mmol) Natriumcarbonat wurden in einer Mischung aus 37 ml Toluol, 15 ml Wasser und 8 ml Ethanol gelöst. Unter Stickstoff wurden 129 mg Tetrakis(triphenylphosphin)palladium(0) zugegeben und 16 Stunden zum Rückfluß erhitzt (Badtemperatur 110 °C).
Zur Aufarbeitung wurden 60 ml Ether zugegeben und dreimal mit je 60 ml Kaliumhydroxidlösung (0,5 M) gewaschen. Die organische Lösung wurde dreimal mit je 23 ml Salzsäure (5 m%) extrahiert, die vereinigten sauren Phasen mit 10 ml Ether gewaschen und mit 14 ml Natronlauge (32 m%) alkalisch gestellt (pH ca. 12). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,32 g Rohbase (86,0 % der Theorie) erhalten, aus der nach Beispiel 1 (3. Stufe) mit Chlortrimethylsilan/Wasser in 2-Butanon 1,11 g (63,9 % der Theorie) [2-(3'-Isopropoxybiphenyl-2-yl)ethyl]dimethylamin-Hydrochlorid mit einem Schmelzpunkt von 164 °C erhalten wurden.

Beispiel 35:

[2-(4'-Chlor-3'-methoxybiphenyl-2-yl)ethyl]dimethylamin-Hydrochlorid

1,20 g (5,43 mmol) des nach Beispiel 34 (1. Stufe) hergestellten Dimethyl-2-(2-dimethylaminoethyl) benzolboronat , 1,80 g (8,14 mmol) 5-Brom-2-chloranisol und 1,92 g (18,1 mmol) Natriumcarbonat wurden in einer Mischung aus 37 ml Toluol, 15 ml Wasser und 8 ml Ethanol gelöst. Unter Stickstoff wurden 129 mg Tetrakis(triphenylphosphin)palladium(0) zugegeben und 16 Stunden zum Rückfluß erhitzt (Badtemperatur 110 °C).
Zur Aufarbeitung wurden 60 ml Ether zugegeben und dreimal mit je 60 ml Kaliumhydroxidlösung (0,5 M) gewaschen. Die organische Lösung wurde dreimal mit je 23 ml Salzsäure (5 m%) extrahiert, die vereinigten sauren Phasen mit 10 ml Ether gewaschen und mit 14 ml Natronlauge (32 m%) alkalisch gestellt (pH ca. 12). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,43 g Rohbase (90,7 % der Theorie) erhalten, aus der nach Beispiel 1 (3. Stufe) mit Chlortrimethylsilan/Wasser in 2-Butanon 1,34 g (75,6 % der Theorie) [2-(4'-Chlor-3'-methoxybiphenyl-2-yl)ethyl]dimethylamin-Hydrochlorid mit einem Schmelzpunkt von 227 °C erhalten wurden.

Beispiel 36:

4-Chlor-2'-(2-dimethylaminethyl)biphenyl-3-ol-Hydrochlorid

0,58 g (2,01 mmol) der Base des nach Beispiel 35 hergestellten [2-(4'-Chlor-3'-methoxybiphenyl-2-yl)ethyl] dimethylamin-Hydrochlorid wurden mit 58 ml Bromwasserstofflösung (48 m% in Wasser) für zwei Stunden zum Rückfluß erhitzt (Badtemperatur 145 °C).
Zur Aufarbeitung wurde der Ansatz in 700 ml Natriumhydrogencarbonatlösung (1 M) gegossen (pH 7-8), dreimal mit je

100 ml Ether extrahiert, die vereinigten organischen Extrakte über wasserfreiem Magnesiumsulfat getrocknet, filtriert und am Rotationsverdampfer (500 bis 10 mbar) eingeengt. Es wurden 0,54 g Rohbase (98,0 % der Theorie) erhalten, aus der nach Beispiel 1 (3. Stufe) mit Chlortrimethylsilan/Wasser in 2-Butanon 0,51 g (93,5 % der Theorie) 4-Chlor-2'-(2-dimethylaminethyl)biphenyl-3-ol-Hydrochlorid mit einem Schmelzpunkt von 164 °C erhalten wurden.

Beispiel 37:

Dimethyl-(3'-nitrobiphenyl-2-ylmethyl)amin-Hydrochlorid

1,04 g (6,20 mmol) 3-Nitrobenzolboronsäure, 1,21 g (5,64 mmol) des nach Beispiel 1 (2. Stufe) hergestellten (2-Brombenzyl)dimethylamin und 1,99 g (18,8 mmol) Natriumcarbonat wurden in einer Mischung aus 40 ml Toluol, 16 ml Wasser und 8 ml Ethanol gelöst. Unter Stickstoff wurden 134 mg Tetrakis(triphenylphosphin)palladium(0) zugegeben und 16 Stunden zum Rückfluß erhitzt (Badtemperatur 110 °C).
Zur Aufarbeitung wurden 65 ml Ether zugegeben und dreimal mit je 65 ml Kaliumhydroxidlösung (0,5 M) gewaschen. Die organische Lösung wurde dreimal mit je 25 ml Salzsäure (5 m%) extrahiert, die vereinigten sauren Phasen mit 10 ml Ether gewaschen und mit 15 ml Natronlauge (32 m%) alkalisch gestellt (pH ca. 12). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 714 mg Rohbase (49,3 % der Theorie) erhalten, die auf eine 3 x 25 cm Säule, gefüllt mit Kieselgel, gegeben wurden. Die Elution mit Ether/n-Hexan 1:3 (V:V) ergab 330 mg Base, aus der nach Beispiel 1 (3. Stufe) mit Chlortrimethylsilan/Wasser in 2-Butanon 262 mg (15,9 % der Theorie) Dimethyl-(3'-nitrobiphenyl-2-ylmethyl)amin-Hydrochlorid mit einem Schmelzpunkt von 147 °C erhalten wurden.

Beispiel 38:

4-Amino-2'-dimethylaminomethylbiphenyl-3-ol-Dihydrochlorid

2,40 g (6,17 mmol) der Base des nach Beispiel 22 hergestellten N-(2'-Dimethyfaminomethyl-3-trifluormethoxybiphenyl-4-yl)acetamid-Hydrochlorid (22) wurden mit 110 ml Bromwasserstofflösung (33 m% in Eisessig) für sechs Stunden zum Rückfluß erhitzt (Badtemperatur 160 °C).
Zur Aufarbeitung wurde der Ansatz in 1000 ml Ether gegossen und der Überstand abdekantiert. Der Rückstand wurde in Wasser gelöst, dreimal mit je 20 ml Ether gewaschen, mit Natriumhydrogencarbonatlösung (1 M) auf pH 7-8 eingestellt, dreimal mit je 40 ml Ether extrahiert, die vereinigten organischen Extrakte über wasserfreiem Magnesiumsulfat getrocknet, filtriert und am Rotationsverdampfer (500 bis 10 mbar) eingeengt. Es wurden 1,94 g Rohbase (130 % der Theorie) erhalten, die auf eine 3 x 30 cm Säule, gefüllt mit Kieselgel, gegeben wurden. Die Elution mit Ether/n-Hexan 2:1 (V:V) ergab neben 1.72 g weitgehend unveränderten Edukts 178 mg Rohbase, aus der nach Beispiel 1 (3. Stufe) mit Chlortrimethylsilan/Wasser in 2-Butanon 177 mg (7,5 % der Theorie) 4-Amino-2'-dimethylaminomethylbiphenyl-3-ol-Dihydrochlorid erhalten wurden, das sich beim Erhitzen ab 120 °C zersetzt.

Beispiel 39:

(3',5'-Difluorbiphenyl-2-ylmethyl)dimethylamin-Hydrochlorid

1,71 g (8,26 mmol) des nach Beispiel 18 (1. Stufe) hergestellten Dimethyl-2-(dimethylaminomethyl) benzolboronat , 1,06 g (5,51 mmol) Brom-3,5-difluorbenzol und 1,94 g (18,3 mmol) Natriumcarbonat wurden in einer Mischung aus 38 ml Toluol, 15 ml Wasser und 7,5 ml Ethanol gelöst. Unter Stickstoff wurden 131 mg Tetrakis (triphenylphosphin)palladium(0) zugegeben und 16 Stunden zum Rückfluß erhitzt (Badtemperatur 110 °C).
Zur Aufarbeitung wurden 60 ml Ether zugegeben und dreimal mit je 60 ml Kaliumhydroxidlösung (0,5 M) gewaschen. Die organische Lösung wurde dreimal mit je 24 ml Salzsäure (5 m%) extrahiert, die vereinigten sauren Phasen mit 25 ml Ether gewaschen und mit 14 ml Natronlauge (32 m%) alkalisch gestellt (pH ca. 12). Es wurde dreimal mit je 25 ml Ether extrahiert, die vereinigten organischen Phasen über wasserfreiem Magnesiumsulfat getrocknet, filtriert und am Rotationsverdampfer (500 - 10 mbar) eingeengt. Es wurden 1,19 g Rohbase (87,6 % der Theorie) erhalten, die auf eine 3 x 25 cm Säule, gefüllt mit Kieselgel, gegeben wurden. Die Elution mit Ether/n-Hexan 1:3 (V:V) ergab 990 mg Base, aus der nach Beispiel 1 (3. Stufe) mit Chlortrimethylsilan/Wasser in 2-Butanon 1,06 g (37,2 % der Theorie) (3',5'-Difluorbiphenyl-2-ylmethyl)dimethylamin-Hydrochlorid erhalten wurden, das sich beim Erhitzen ab 190 °C zersetzt.

Beispiel 40:

(2',5'-Dimethoxybiphenyl-2-ylmethyl)dimethylamin-Hydrochlorid

1,61 g (7,79 mmol) des nach Beispiel 18 (1. Stufe) hergestellten Dimethyl-2-(dimethylaminomethyl) benzolboronat , 1,13 g (5,20 mmol) 2-Brom-1,4-dimethoxy-benzol und 1,83 g (17,3 mmol) Natriumcarbonat wurden in einer Mischung aus 35 ml Toluol, 14 ml Wasser und 7 ml Ethanol gelöst. Unter Stickstoff wurden 123 mg Tetrakis (triphenylphosphin)palladium(0) zugegeben und 16 Stunden zum Rückfluß erhitzt (Badtemperatur 110 °C).
Zur Aufarbeitung wurden 55 ml Ether zugegeben und dreimal mit je 55 ml Kaliumhydroxidlösung (0,5 M) gewaschen. Die organische Lösung wurde dreimal mit je 22 ml Salzsäure (5 m%) extrahiert, die vereinigten sauren Phasen mit 10 ml Ether gewaschen und mit 13 ml Natronlauge (32 m%) alkalisch gestellt (pH ca. 12). Es wurde dreimal mit je 20 ml Ether extrahiert, die vereinigten organischen Phasen über wasserfreiem Magnesiumsulfat getrocknet, filtriert und am Rotationsverdampfer (500 - 10 mbar) eingeengt. Es wurden 770 mg Rohbase (61,0 % der Theorie) erhalten, aus der nach Beispiel 1 (3. Stufe) mit Chlortrimethylsilan/Wasser in 2-Butanon 777 mg (49,7 % der Theorie) (2',5'-Dimethoxybiphenyl-2-ylmethyl)dimethylamin-Hydrochlorid mit einem Schmelzpunkt von 169 °C erhalten wurden

**Beispiel 41:**

2'-Dimethylaminomethyl-5-trifluormethoxybiphenyl-2-ylamin-Dihydrochlorid

6,24 g (30,1 mmol) des nach Beispiel 18 (1. Stufe) hergestellten Dimethyl-2-(dimethylaminomethyl)benzolboronat, 5,14 g (20,1 mmol) 2-Brom-4-trifluormethoxyphenylamin und 7,09 g (66,9 mmol) Natriumcarbonat wurden in einer Mischung aus 140 ml Toluol, 55 ml Wasser und 27 ml Ethanol gelöst. Unter Stickstoff wurden 476 mg Tetrakis(triphenylphosphin)palladium(0) zugegeben und 16 Stunden zum Rückfluß erhitzt (Badtemperatur 110 °C).
Zur Aufarbeitung wurden 220 ml Ether zugegeben und dreimal mit je 220 ml Kaliumhydroxidlösung (0,5 M) gewaschen. Die organische Lösung wurde dreimal mit je 90 ml Salzsäure (5 m%) extrahiert, die vereinigten sauren Phasen mit 90 ml Ether gewaschen und mit 52 ml Natronlauge (32 m%) alkalisch gestellt (pH ca. 12). Es wurde dreimal mit je 90 ml Ether extrahiert, die vereinigten organischen Phasen über wasserfreiem Magnesiumsulfat getrocknet, filtriert und am Rotationsverdampfer (500 - 10 mbar) eingeengt. Es wurden 3,46 g Rohbase (55,5 % der Theorie) erhalten, die auf eine 4 x 30 cm Säule, gefüllt mit Kieselgel, gegeben wurden. Die Elution mit Ether/n-Hexan 1:1 (V:V) ergab 1,73 g Base (27,8 % der Theorie). Aus 316 mg dieser Base wurden nach Beispiel 1 (3. Stufe) mit Chlortrimethylsilan/Wasser in 2-Butanon 389 mg 2'-Dimethylaminomethyl-5-trifluormethoxybiphenyl-2-ylamin-Dihydrochlorid mit einem Schmelzpunkt von 125 °C erhalten wurden.

Beispiel 42:

N-(2'-Dimethylaminomethyl-5-trifluormethoxybiphenyl-2-yl)acetamid-Hydrochlorid

1,42 g (4,56 mmol) der Base des nach Beispiel 41 hergestellten 2'-Dimethylaminomethyl-5-trifluormethoxybiphenyl-2-ylamins wurden mit 80 ml Bromwasserstofflösung (33 m% in Eisessig) 24 Stunden erhitzt (Badtemperatur 140 °C).
Zur Aufarbeitung wurde der Ansatz in 800 ml Ether gegossen und der Überstand abdekantiert. Der Rückstand wurde in Wasser gelöst, dreimal mit je 50 ml Ether gewaschen, mit Kaliumhydroxidlösung (1 M) alkalisch gestellt (pH > 12), dreimal mit je 50 ml Ether extrahiert, die vereinigten organischen Extrakte über wasserfreiem Magnesiumsulfat getrocknet, filtriert und am Rotationsverdampfer (500 bis 10 mbar) eingeengt. Es wurden 1,94 g Rohbase (130 % der Theorie) erhalten, die auf eine 3 x 30 cm Säule, gefüllt mit Kieselgel, gegeben wurden. Die Elution mit Ether ergab 940 mg Base (85,1 % der Theorie). Aus 303 mg dieser Base wurden nach Beispiel 1 (3. Stufe) mit Chlortrimethylsilan/Wasser in 2-Butanon 274 mg N-(2'-Dimethylaminomethyl-5-trifluormethoxybiphenyl-2-yl)acetamid-Hydrochlorid mit einem Schmelzpunkt von 115 °C erhalten wurden.

Beispiel 43:

3,5-Dichlor-2'-dimethylaminomethyl-biphenyl-4-ylamin Hydrochlorid

6,14 g (29,7 mmol) des nach Beispiel 18 (1. Stufe) hergestellten Dimethyl-2-(dimethylaminomethyl)benzolboronat, 10,0 g (41,5 mmol) 4-Brom-2,6-dichloranilin und 10,5 g (98,7 mmol) Natriumcarbonat wurden in einer Mischung aus 100 ml Toluol, 40 ml Wasser und 20 ml Ethanol gelöst. Unter Stickstoff wurden 354 mg Tetrakis(triphenylphosphin) palladium(0) zugegeben und 16 Stunden zum Rückfluß erhitzt (Badtemperatur 110 °C).

Zur Aufarbeitung wurden 200 ml Ether zugegeben und dreimal mit je 130 ml Kaliumhydroxidlösung (0,5 M) gewaschen. Die organische Lösung wurde dreimal mit je 90 ml Salzsäure (5 m%) extrahiert, die vereinigten sauren Phasen mit 50 ml Ether gewaschen und mit 45 ml Natronlauge (32 m%) alkalisch gestellt (pH ca. 12). Es wurde dreimal mit je 100 ml Ether extrahiert, die vereinigten organischen Phasen über wasserfreiem Magnesiumsulfat getrocknet, filtriert und am Rotationsverdampfer (500 - 10 mbar) eingeengt. Es wurden 6,88 g Rohbase (78,7 % der Theorie) erhalten. Aus 765 mg dieses Rohprodukts wurde nach Beispiel 1 (3. Stufe) mit Chlortrimethylsilan/Wasser in 2-Butanon 470 mg (54,8 % der Theorie) 3,5-Dichlor-2'-dimethylaminomethylbiphenyl-4-ylamin-Hydrochlorid erhalten.

Pharmakologische Untersuchungen

Writhing-Test an der Maus

Die Untersuchung auf analgetische Wirksamkeit wurde im Phenylchinoninduzierten Writhing an der Maus (modifiziert nach I.C. Hendershot, J. Forsaith, J. Pharmacol. Exp. Ther. 125, 237-240 (1959)) durchgeführt. Dazu wurden männliche NMRI-Mäuse im Gewicht von 25-30 g verwendet. Gruppen von 10 Tieren pro Substanzdosis erhielten 10 Minuten nach intravenöser Gabe der Prüfsubstanzen 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die nur physiologische Kochsalzlösung erhalten. Alle Substanzen wurden in der Standarddosierung von 10 mg/kg getestet. Die prozentuale Hemmung (%Hemmung) der Writhingreaktion durch eine Substanz wurde nach folgender Formel berechnet:

$$\% \text{ Hemmung} = 100 - \frac{\text{Writhingreaktionen der behandelten Tiere}}{\text{Writhingreaktionen der Kontrolltiere}} * 100$$

Für einige Substanzen wurde aus der dosisabhängigen Abnahme der Writhingreaktionen im Vergleich zu parallel untersuchten Phenylchinon-Kontrollgruppen mittels Regressionsanalyse (Auswerteprogramm Martens EDV Service, Eckental) die $ED_{50}$-Werte mit 95 % Vertrauensbereich der Writhingreaktion berechnet.
Alle untersuchten erfindungsgemäßen Verbindungen zeigten eine ausgeprägte analgetische Wirkung. Die Ergebnisse sind in der nachfolgenden Tabelle zusammengefaßt.

Tabelle 1: Writhing-Test an der Maus

| Beispiel | %Hemmung der Writhingreaktion bei 10 mg/kg intravenös | $ED_{50}$ (mg/kg intravenös) |
|---|---|---|
| (1) | 53 | |
| (2) | 89 | 4,55 |
| (3) | 100 | 0,24 |
| (4) | 75 | 3,95 |
| (5) | 68 | 4, 31 |
| (6) | 88 | 4,26 |
| (7) | 90 | 2,55 |
| (8) | 67 | 7,15 |
| (9) | 89 | 2,32 |
| (10) | 100 | 1,70 |
| (11) | 74 | |
| (12) | 89 | 1,74 |
| (13) | 81 | 5,30 |

(fortgesetzt)

| Beispiel | %Hemmung der Writhingreaktion bei 10 mg/kg intravenös | $ED_{50}$ (mg/kg intravenös) |
|---|---|---|
| (14) | 100 | 2,28 |
| (15) | 52 | 3,66 |
| (16) | 89 | 4, 59 |
| (17) | 90 | |
| (18) | 83 | |
| (19) | 86 | |
| (20) | 83 | 4,99 |
| (21) | 53 | 6, 78 |
| (22) | 76 | 6,05 |
| (23) | 97 | |
| (24) | 99 | 2,27 |
| (25) | 74 | |
| (26) | 100 | 0,75 |
| (27) | 89 | |
| (28) | 90 | |
| (29) | 83 | 5,71 |
| (30) | 89 | |
| (31) | 94 | |
| (32) | 98 | |
| (33) | 89 | |
| (34) | 80 | |
| (35) | 88 | |
| (36) | 93 | |
| (37) | 60 | |
| (38) | 65 | |
| (39) | 80 | |
| (40) | 57 | |
| (41) | 56 | |
| (42) | 44 | |
| (43) | 99 | |

## Patentansprüche

**1.** Substituierte 2-Aminoalkylbiphenyl-Derivate der allgemeinen Formel I

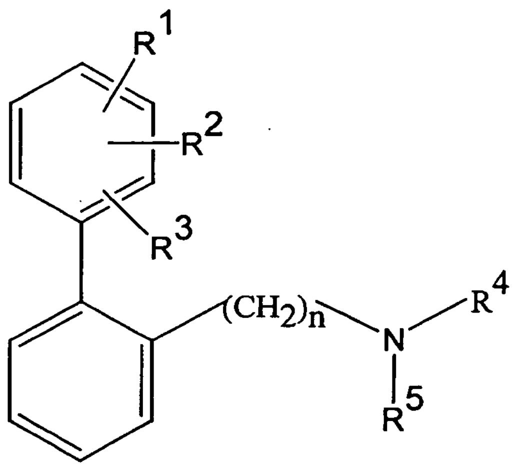

I,

worin

n = 1 oder 2 ist,
der Rest $R^1$ für CN, $NO_2$, $SO_2CH_3$, $SO_2CF_3$, O-Aryl, O-$C_{1-6}$-Alkylen-Aryl, $NR^6R^7$, einen Aryl-, einen Acetyl, einen Acetamidyl-, oder für einen über eine $C_{1-6}$-Alkylen-Gruppe gebundenen Aryl-Rest steht,
der Rest $R^2$ für H, F, Cl, Br, CN, $NO_2$, CHO, $SO_2CH_3$, $SO_2CF_3$, $OR^6$, $NR^6R^7$, einen $C_{1-6}$-Alkyl-, einen Aryl-, einen Acetyl, einen Acetamidyl-, einen Benzoyl- oder für einen über eine $C_{1-6}$-Alkylen-Gruppe gebundenen Aryl-Rest steht,
oder $R^1$ und $R^2$ zusammen jeweils die Gruppe $OCH_2CH_2O$, CH=CHO, CH=C($CH_3$)O oder CH=CHNH bedeuten,
der Rest $R^3$ für H, F, Cl, Br, CN, $NO_2$, CHO, $SO_2CH_3$, $SO_2CF_3$, $OR^6$, $NR^6R^7$, einen $C_{1-6}$-Alkyl-, einen Aryl-, einen Acetyl, einen Acetamidyl-, einen Benzoyl- oder für einen über eine $C_{1-6}$-Alkylen-Gruppe gebundenen Aryl-Rest steht,
die Reste $R^4$, $R^5$, gleich oder verschieden, für H oder für einen $C_{1-6}$ Alkyl-Rest stehen,
die Reste $R^6$, $R^7$, gleich oder verschieden, für H, einen $C_{1-6}$-Alkyl-, einen Aryl- oder für einen über eine $C_{1-6}$-Alkylen-Gruppe gebundenen Aryl-Rest stehen,
wobei
die Alkylreste jeweils auch mindestens einfach, gleich oder verschieden, mit Halogen und/oder einer Hydroxy-Gruppe substituiert sein können, und
die Aryl-Reste jeweils auch mindestens einfach mit einem OH-, einem Halogen-, einem $CF_3$-, einem $C_{1-6}$-Alkyl-, einem $C_{1-6}$-Alkoxy-, einem $C_{1-7}$-Cycloalkoxy-, einem $C_{3-7}$-Cycloalkyl-, einem $C_{2-6}$-Alkylen- oder Phenyl-Rest substituierte Phenyle oder Naphthyl-Reste bedeuten können,
in Form ihrer Basen und/oder Salze von physiologisch verträglichen Säuren, wobei die Verbindungen
4-Chlor-2'-dimethylaminomethylbiphenyl-2-carbonitril und
4-(2'-N,N-dimethylaminomethylphenyl)-2-fluoranilin
ausgenommen sind.

2. Substituierte 2-Aminoalkylbiphenyl-Derivate gemäß Anspruch 1, **dadurch gekennzeichnet, daß** die Reste $R^2$ und/oder $R^3$ für einen $C_{1-3}$-Alkylrest stehen und die übrigen Substituenten sowie n die Bedeutung gemäß der allgemeinen Formel I haben.

3. Substituierte 2-Aminoalkylbiphenyl-Derivate gemäß Anspruch 1 oder 2, **dadurch gekennzeichnet, daß** die Reste $R^1$, $R^2$ und/oder $R^3$ für einen über eine $C_{1-3}$-Alkylen-Gruppe gebundenen Aryl-Rest stehen und die übrigen Substituenten sowie n die Bedeutung gemäß der allgemeinen Formel I haben.

4. Substituierte 2-Aminoalkylbiphenyl-Derivate gemäß einem oder mehreren der Ansprüche 1 bis 3, **dadurch gekennzeichnet, daß** die Reste $R^4$ und/oder $R^5$ für einen $C_{1-3}$-Alkylrest stehen und die übrigen Substituenten sowie n die Bedeutung gemäß der allgemeinen Formel I haben.

5. Substituierte 2-Aminoalkylbiphenyl-Derivate gemäß einem oder mehreren der Ansprüche 1 bis 4, **dadurch gekenn-**

**zeichnet, daß** die Reste $R^6$ und/oder $R^7$ für einen $C_{1-3}$-Alkylrest stehen und die übrigen Substituenten sowie n die Bedeutung gemäß der allgemeinen Formel I haben.

6. Substituierte 2-Aminoalkylbiphenyl-Derivate gemäß einem oder mehreren der Ansprüche 1 bis 5, **dadurch gekennzeichnet, daß** die Reste $R^6$ und/oder $R^7$ für einen über eine $C_{1-3}$-Alkylen-Gruppe gebundenen Aryl-Rest stehen und die übrigen Substituenten sowie n die Bedeutung gemäß der allgemeinen Formel I haben.

7. Substituierte 2-Aminoalkylbiphenyl-Derivate gemäß Anspruch 1:

Dimethyl-[2-(2-methylbenzofuran-4-yl)benzyl]amin und das entsprechende Hydrochlorid
Dimethyl-(4'-nitro-3'-trifluormethylbiphenyl-2-ylmethyl)-amin und das entsprechende Hydrochlorid
N-(2'-Dimethylaminomethyl-3-trifluormethoxybiphenyl-4-yl)acetamid und das entsprechende Hydrochlorid
[2-(1*H*-Indol-5-yl)benzyl]dimethylamin und das entsprechende Hydrochlorid
(4'-Methansulfonylbiphenyl-2-ylmethyl)dimethylamin und das entsprechende Hydrochlorid
1-[2'-(2-Dimethylaminoethyl)biphenyl-3-yl]ethanon und das entsprechende Hydrochlorid
Dimethyl-(3'-nitrobiphenyl-2-ylmethyl)amin und das entsprechende Hydrochlorid
4-Amino-2'-dimethylaminomethylbiphenyl-3-ol und das entsprechende Dihydrochlorid
2'-Dimethylaminomethyl-5-trifluormethoxybiphenyl-2-ylamin und das entsprechende Dihydrochlorid
N-(2'-Dimethylaminomethyl-5-trifluormethoxybiphenyl-2-yl)acetamid und das entsprechende Hydrochlorid
3,5-Dichlor-2'-dimethylaminomethyl-biphenyl-4-ylamin und das entsprechende Hydrochlorid.

8. Verfahren zur Herstellung von substituierten 2-Aminoalkyl-biphenyl-Derivaten der allgemeinen Formel I nach einem oder mehreren der Ansprüche 1 bis 7, **dadurch gekennzeichnet, daß** man die Verbindungen der allgemeinen Formel II

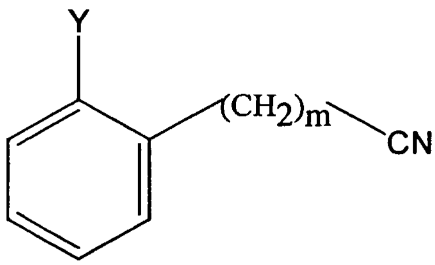

II,

worin Y = Cl, Br oder I und m = 0 oder 1 bedeuten, in Lösung mit einem Reduktionsmittel zu Verbindungen der allgemeinen Formel III, worin n = 1 oder 2 bedeutet, reduziert

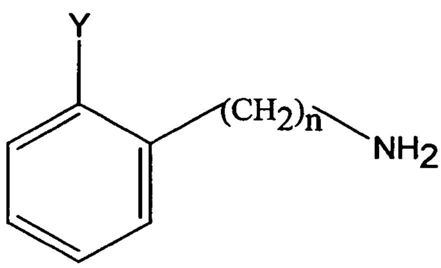

III

und diese nach üblichen Methoden reinigt und isoliert,
die Verbindungen der allgemeinen Formel III in Gegenwart eines Reduktionsmittels mit aliphatischen $C_{1-6}$-Aldehyden zu Verbindungen der allgemeinen Formel IV umsetzt

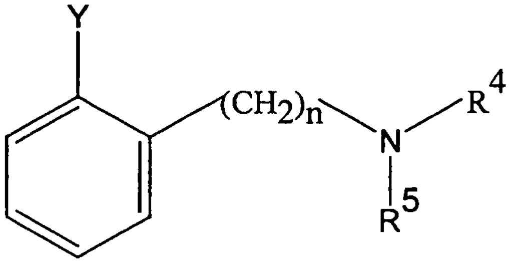

IV,

worin $R^4$ und $R^5$ die Bedeutung gemäß der allgemeinen Formel I nach Anspruch 1 haben, und diese nach üblichen Methoden reinigt und isoliert,
die Verbindungen der allgemeinen Formel IV durch Halogen-Metallaustausch und anschließende Reaktion mit einem Borsäureester bei einer Temperatur ≤ 0 °C zu Verbindungen der allgemeinen Formel V, worin R einen $C_{1-6}$-Alkyl-Rest bedeutet, umsetzt

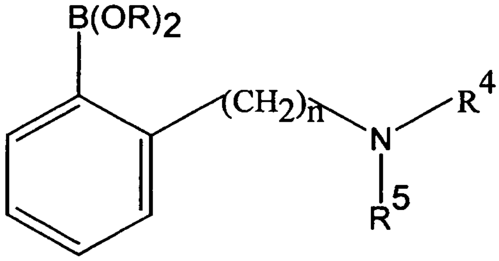

V,

und diese nach üblichen Methoden isoliert und reinigt oder ohne weitere Aufreinigung im folgenden Reaktionsschritt einsetzt,
die Verbindungen der allgemeinen Formel V mit wäßrigen Säuren zu Verbindungen der allgemeinen Formel VI umsetzt

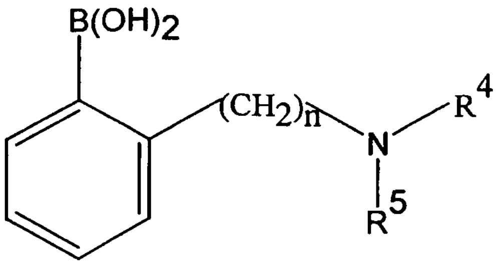

VI

und diese nach üblichen Methoden reinigt und isoliert,
die Verbindungen der allgemeinen Formel V oder VI in einer Übergangsmetall-katalysierten Reaktion in einem aliphatischen Ether, einem Kohlenwasserstoff, einem Alkohol, einem chlorierten Kohlenwasserstoff, Wasser oder Gemischen aus diesen Lösungsmitteln bei Temperaturen zwischen 20 und 150 °C mit Verbindungen der allgemeinen Formel VII

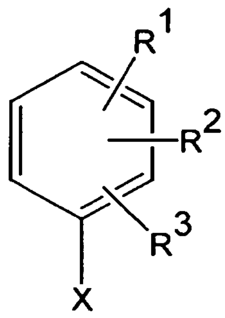

VII,

worin X = Cl, Br, 1 oder $OSO_2C_pF_{(2p+1)}$ bedeutet und die Reste $R^1$ bis $R^3$ die Bedeutung gemäß der allgemeinen Formel I nach Anspruch 1 haben, zu Verbindungen der allgemeinen Formel I umsetzt
und diese nach üblichen Methoden reinigt und isoliert, oder
die Verbindungen der allgemeinen Formel VIII oder IX

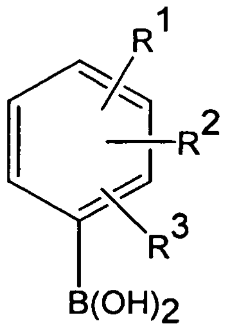

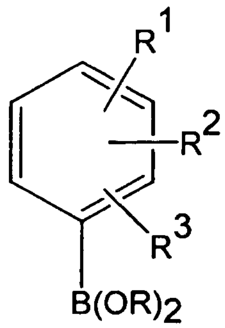

VIII IX,

worin $R^1$ bis $R^3$ die Bedeutung gemäß der allgemeinen Formel I nach Anspruch 1 haben, in einer Übergangsmetall-katalysierten Reaktion in einem aliphatischen Ether, einem Kohlenwasserstoff, einem Alkohol, einem chlorierten Kohlenwasserstoff, Wasser oder Gemischen aus diesen Lösungsmitteln bei Temperaturen zwischen 20 und 150 °C mit Verbindungen der allgemeinen Formel III oder IV zu Verbindungen der allgemeinen Formel I umsetzt und diese nach üblichen Methoden reinigt und isoliert.

**9.** Verfahren gemäß Anspruch 8, **dadurch gekennzeichnet, daß** man die Verbindungen der allgemeinen Formel II mit Lithiumaluminiumhydrid und/oder Diisobutylaluminiumhydrid reduziert.

**10.** Verfahren gemäß Anspruch 8 oder 9, **dadurch gekennzeichnet, daß** man die Verbindungen der allgemeinen Formel III in Gegenwart von Ameisensäure und/oder Natriumborhydrid mit aliphatischen $C_{1-6}$-Aldehyden umsetzt.

**11.** Verfahren gemäß einem oder mehreren der Ansprüche 8 bis 10, **dadurch gekennzeichnet, daß** der Halogen-Metallaustausch mit Magnesium und/oder Butyllithium durchgeführt wird.

**12.** Verfahren gemäß einem oder mehreren der Ansprüche 8 bis 11, **dadurch gekennzeichnet, daß** der Borsäureester einTrialkylborat, bevorzugt Trimethylborat ist.

**13.** Verfahren gemäß einem oder mehreren der Ansprüche 8 bis 12, **dadurch gekennzeichnet, daß** die Verbindungen der allgemeinen Formel V mit Salzsäure zu Verbindungen der allgemeinen Formel VI umgesetzt werden.

**14.** Verfahren gemäß einem oder mehreren der Ansprüche 8 bis 13, **dadurch gekennzeichnet, daß** die Verbindungen der allgemeinen Formel V oder VI in einer durch Palladium(0)-Verbindungen und/oder durch Palladium(II)-Salze,

bevorzugt durch Tetrakis(triphenylphosphin)palladium, Bis(dibenzylidenaceton)palladium, elementares Palladium auf Aktivkohle, Palladium(II)chlorid und/oder Palladium(II)acetat katalysierten Reaktion umgesetzt werden.

**15.** Verfahren gemäß einem oder mehreren der Ansprüche 8 bis 13, **dadurch gekennzeichnet, daß** die Verbindungen der allgemeinen Formel VIII oder IX in einer durch Palladium(0)-Verbindungen oder durch Palladium(II)-Salze, bevorzugt durch Tetrakis(triphenylphosphin)palladium, Bis(dibenzylidenaceton)palladium, elementares Palladium auf Aktivkohle, Palladium(II)chlorid und/oder Palladium(II)acetat katalysierten Reaktion umgesetzt werden.

**16.** Verfahren gemäß einem oder mehreren der Ansprüche 8 bis 15, **dadurch gekennzeichnet, daß** die Übergangsmetall-katalysierte Reaktion in 1,4-Dioxan, Tetrahydrofuran, Toluol, Hexan, Ethanol, Isopropanol, Chloroform, Dichlormethan, Wasser oder Gemischen dieser Lösungsmittel durchgeführt wird.

**17.** Arzneimittel enthaltend als pharmazeutischen Wirkstoff wenigstens ein substituiertes 2-Aminoalkylbiphenyl-Derivat der allgemeinen Formel I

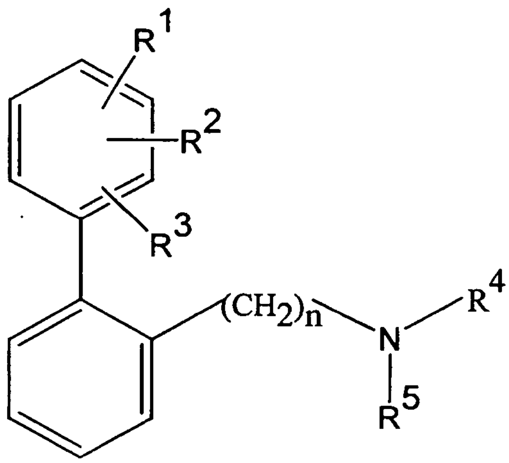

I

worin

n = 1 oder 2 ist,
die Reste $R^1$, $R^2$, $R^3$, gleich oder verschieden für H, F, Cl, Br, CN, $NO_2$, CHO, $SO_2CH_3$, $SO_2CF_3$, $OR^6$, $NR^6R^7$, einen $C_{1-6}$-Alkyl-, einen Aryl-, einen Acetyl, einen Acetamidyl-, einen Benzoyl- oder für einen über eine $C_{1-6}$-Alkylen-Gruppe gebundenen Aryl-Rest stehen,
oder $R^1$ und $R^2$ zusammen jeweils die Gruppe $OCH_2O$ $OCH_2CH_2O$, CH=CHO, $CH=C(CH_3)O$ oder CH=CHNH bedeuten,
die Reste $R^4$, $R^5$, gleich oder verschieden, für H oder für einen C1-6 Alkyl-Rest stehen,
die Reste $R^6$, $R^7$, gleich oder verschieden, für H, einen $C_{1-6}$-Alkyl-, einen Aryl- oder für einen über eine $C_{1-6}$-Alkylen-Gruppe gebundenen Aryl-Rest stehen,
wobei die Alkylreste jeweils auch mindestens einfach, gleich oder verschieden, mit Halogen und/oder einer Hydroxy-Gruppe substituiert sein können, und
die Aryl-Reste jeweils auch mindestens einfach mit einem OH-, einem Halogen-, einem $CF_3$-, einem $C_{1-6}$-Alkyl-, einem $C_{1-6}$-Alkoxy-, einem $C_{1-7}$-Cycloalkoxy-, einem $C_{3-7}$-Cycloalkyl-, einem $C_{2-6}$-Alkylen- oder Phenyl-Rest substituierte Phenyle oder Naphthyl-Reste bedeuten können,
in Form seiner Base und/oder eines Salzes einer physiologisch verträglichen Säure und gegebenfalls weitere Wirkstoffe und/oder Hilfsstoffe.

**18.** Arzneimittel gemäß Anspruch 17 enthaltend als pharmazeutischen Wirkstoff wenigstens ein substituiertes 2-Aminoalkylbiphenyl-Derivat ausgewählt aus der Gruppe umfassend folgende Verbindungen:

(3'-Methoxybiphenyl-2-ylmethyl)dimethylamin und das entsprechende Hydrochlorid,
(4'-Chlorbiphenyl-2-ylmethyl)dimethylamin und das entsprechende Hydrochlorid,

2'-Dimethylaminomethylbiphenyl-3-ol und das entsprechende Hydrochlorid,
(2'-Methoxybiphenyl-2-ylmethyl)dimethylamin und das entsprechende Hydrochlorid,
(3'-Chlorbiphenyl-2-ylmethyl)dimethylamin und das entsprechende Hydrochlorid,
(2'-Fluorbiphenyl-2-ylmethyl)dimethylamin und das entsprechende Hydrochlorid,
(3'-Fluorbiphenyl-2-ylmethyl)dimethylamin und das entsprechende Hydrochlorid,
(4'-Fluorbiphenyl-2-ylmethyl)dimethylamin und das entsprechende Hydrochlorid,
(3'-Chlor-4'-fluorbiphenyl-2-ylmethyl)dimethylamin und das entsprechende Hydrochlorid,
(3'-Methoxybiphenyl-2-ylethyl)dimethylamin und das entsprechende Hydrochlorid,
Dimethyl-[2-(2-methylbenzofuran-4-yl)benzyl]amin und das entsprechende Hydrochlorid,
2'-Dimethylaminomethytbiphenyl-2-carbaldehyd Hydrochlorid,
(3'-Difluormethylbiphenyl-2-ylmethyl)dimethylamin und das entsprechende Hydrochlorid,
2'-Dimethylaminomethylbiphenyl-3-carbaldehyd und das entsprechende Hydrochlorid,
Biphenyl-2-ylmethyldimethylamin Hydrochlorid,
(3',4'-Dichlorbiphenyl-2-ylmethyl)dimethylamin und das entsprechende Hydrochlorid,
(3',5'-Dichlorbiphenyl-2-ylmethyl)dimethylamin und das entsprechende Hydrochlorid,
Dimethyl-(4'-nitro-3'-trifluormethylbiphenyl-2-ylmethyl)-amin und das entsprechende Hydrochlorid,
(3',4'-Difluorbiphenyl-2-ylmethyl)dimethylamin und das entsprechende Hydrochlorid,
(4'-Fluor-3'-trifluormethylbiphenyl-2-ylmethyl)dimethyl-amin und das entsprechende Hydrochlorid,
(4'-Chlor-3'-methoxybiphenyl-2-ylmethyl)dimethylamin und das entsprechende Hydrochlorid,
N-(2'-Dimethylaminomethyl-3-trifluormethoxybiphenyl-4-yl)acetamid und das entsprechende Hydrochlorid,
(3'-Isopropoxybiphenyl-2-ylmethyl)dimethylamin und das entsprechende und das entsprechende Hydrochlorid,
2'-(2-Dimethylaminoethyl)biphenyl-3-ol und das entsprechende Hydrochlorid,
4-Chlor-2'-dimethylaminomethylbiphenyl-3-ol und das entsprechende Hydrochlorid,
[2-(1*H*-Indol-5-yl)benzyl]dimethylamin und das entsprechende Hydrochlorid,
(4'-Methansulfonylbiphenyl-2-ylmethyl)dimethylamin und das entsprechende Hydrochlorid,
(2',4'-Dichlorbiphenyl-2-ylmethyl)dimethylamin und das entsprechende Hydrochlorid,
(2',3'-Difluorbiphenyl-2-ylmethyl)dimethylamin und das entsprechende Hydrochlorid,
(2',5'-Difluorbiphenyl-2-ylmethyl)dimethylamin und das entsprechende Hydrochlorid,
(2-Benzo[1,3]dioxol-5-ylbenzyl)dimethylamin und das entsprechende Hydrochlorid,
1-[2'-(2-Dimethylaminoethyl)biphenyl-3-yl]ethanon und das entsprechende Hydrochlorid,
[2-(3',4'-Dimethoxybiphenyl-2-ylethyl]dimethylamin und das entsprechende Hydrochlorid,
[2-(3'-Isopropoxybiphenyl-2-yl)ethyl]dimethylamin und das entsprechende Hydrochlorid,
[2-(4'-Chlor-3'-methoxybiphenyl-2-yl)ethyl]dimethylamin und das entsprechende Hydrochlorid,
4-Chlor-2'-(2-dimethylaminoethyl)biphenyl-3-ol und das entsprechende Hydrochlorid,
Dimethyl-(3'-nitrobiphenyl-2-ylmethyl)amin und das entsprechende Hydrochlorid,
4-Amino-2'-dimethylaminomethylbiphenyl-3-ol und das entsprechende Dihydrochlorid,
(3',5'-Difluorbiphenyl-2-ylmethyl)dimethylamin und das entsprechende Hydrochlorid,
(2',5'-Dimethoxybiphenyl-2-ylmethyl)dimethylamin und das entsprechende Hydrochlorid,
2'-Dimethylaminomethyl-5-trifluormethoxybiphenyl-2-ylamin und das entsprechende Dihydrochlorid,
N-(2'-Dimethylaminomethyl-5-trifluormethoxybiphenyl-2-yl)acetamid und das entsprechende Hydrochlorid sowie
3,5-Dichlor-2'-dimethylaminomethyl-biphenyl-4-ylamin und das entsprechende Hydrochlorid.

**19.** Arzneimittel nach Anspruch 17 oder 18 zur Behandlung/Bekämpfung bei/von Schmerzen, inflammatorischen und allergischen Reaktionen, Depressionen, Drogen- und Alkoholmißbrauch, Gastritis, Diarrhoe, Harninkontinenz, cardiovaskulären Erkrankungen, Atemwegserkrankungen, Husten, seelischen Erkrankungen und/oder Epilepsie.

**20.** Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Bekämpfung von Schmerzen.

**21.** Verwendung wenigstens eines substituierten 2-Aminoalkyl-biphenyl-Derivates der allgemeinen Formel I gemäß Anspruch 17 in Form seiner Basen und/oder eines Salzes von physiologisch verträglichen Säuren zur Herstellung eines Arzneimittels zur Behandlung von inflammatorischen Reaktionen.

**22.** Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von allergischen Reaktionen.

23. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von Depressionen.

24. Verwendung wenigstens eines substituierten 2-Aminoalkyl-biphenyl-Derivates der allgemeinen Formel I gemäß Anspruch 17 in Form seiner Basen und/oder eines Salzes von physiologisch verträglichen Säuren zur Herstellung eines Arzneimittels zur Behandlung von Drogen- und/oder Alkoholmißbrauch.

25. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von Gastritis.

26. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von Diarrhoe.

27. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von Harninkontinenz.

28. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von cardiovaskulären Erkrankungen.

29. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von Atemwegserkrankungen.

30. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säure zur Herstellung eines Arzneimittels zur Behandlung von Husten.

31. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säuren zur Herstellung eines Arzneimittels zur Behandlung von seelischen Erkrankungen.

32. Verwendung wenigstens eines substituierten 2-Aminoalkyl-biphenyl-Derivates der allgemeinen Formel I gemäß Anspruch 17 in Form seiner Base und/oder eines Salzes einer physiologisch verträglichen Säuren zur Herstellung eines Arzneimittels zur Behandlung von Epilepsie.

33. Verwendung gemäß einem der Ansprüche 20-32, **dadurch gekennzeichnet, daß** das substituierte 2-Aminoalkyl-biphenyl-Derivat ausgewählt ist aus der Gruppe umfassend folgende Verbindungen:

(3'-Methoxybiphenyl-2-ylmethyl)dimethylamin und das entsprechende Hydrochlorid,
(4'-Chlorbiphenyl-2-ylmethyl)dimethylamin und das entsprechende Hydrochlorid,
2'-Dimethylaminomethylbiphenyl-3-ol und das entsprechende Hydrochlorid,
(2'-Methoxybiphenyl-2-ylmethyl)dimethylamin und das entsprechende Hydrochlorid,
(3'-Chlorbiphenyl-2-ylmethyl)dimethylamin und das entsprechende Hydrochlorid,
(2'-Fluorbiphenyl-2-ylmethyl)dimethylamin und das entsprechende Hydrochlorid,
(3'-Fluorbiphenyl-2-ylmethyl)dimethylamin und das entsprechende Hydrochlorid,
(4'-Fluorbiphenyl-2-ylmethyl)dimethylamin und das entsprechende Hydrochlorid,
(3'-Chlor-4'-fluorbiphenyl-2-ylmethyl)dimethylamin und das entsprechende. Hydrochlorid,
(3'-Methoxybiphenyl-2-ylethyl)dimethylamin und das entsprechende Hydrochlorid,
Dimethyl-[2-(2-methylbenzofuran-4-yl)benzyl]amin und das entsprechende Hydrochlorid,
2'-Dimethylaminomethylbiphenyl-2-carbaldehyd Hydrochlorid,
(3'-Difluormethylbiphenyl-2-ylmethyl)dimethylamin und das entsprechende Hydrochlorid,
2'-Dimethylaminomethylbiphenyl-3-carbaldehyd und das entsprechende Hydrochlorid,
Biphenyl-2-ylmethyldimethylamin Hydrochlorid,

(3',4'-Dichlorbiphenyl-2-ylmethyl)dimethylamin und das entsprechende Hydrochlorid,
(3',5'-Dichlorbiphenyl-2-ylmethyl)dimethylamin und das entsprechende Hydrochlorid,
Dimethyl-(4'-nitro-3'-trifluormethylbiphenyl-2-ylmethyl)-amin und das entsprechende Hydrochlorid,
(3',4'-Difluorbiphenyl-2-ylmethyl)dimethylamin und das entsprechende Hydrochlorid,
(4'-Fluor-3'-trifluormethylbiphenyl-2-ylmethyl)dimethyl-amin und das entsprechende Hydrochlorid,
(4'-Chlor-3'-methoxybiphenyl-2-ylmethyl)dimethylamin und das entsprechende Hydrochlorid,
*N*-(2'-Dimethylaminomethyl-3-trifluormethoxybiphenyl-4-yl)acetamid und das entsprechende Hydrochlorid,
(3'-Isopropoxybiphenyl-2-ylmethyl)dimethylamin und das entsprechende und das entsprechende Hydrochlorid,
2'-(2-Dimethylaminoethyl)biphenyl-3-ol und das entsprechende Hydrochlorid,
4-Chlor-2'-dimethylaminomethylbiphenyl-3-ol und das entsprechende Hydrochlorid,
[2-(1*H*-Indol-5-yl)benzyl]dimethylamin und das entsprechende Hydrochlorid,
(4'-Methansulfonylbiphenyl-2-ylmethyl)dimethylamin und das entsprechende Hydrochlorid,
(2',4'-Dichlorbiphenyl-2-ylmethyl)dimethylamin und das entsprechende Hydrochlorid,
(2',3'-Difluorbiphenyl-2-ylmethyl)dimethylamin und das entsprechende Hydrochlorid,
(2',5'-Difluorbiphenyl-2-ylmethyl)dimethylamin und das entsprechende Hydrochlorid,
(2-Benzo[1,3]dioxol-5-ylbenzyl)dimethylamin und das entsprechende Hydrochlorid,
1-[2'-(2-Dimethylaminoethyl)biphenyl-3-yl]ethanon und das entsprechende Hydrochlorid,
[2-(3',4'-Dimethoxybiphenyl-2-ylethyl]dimethylamin und das entsprechende Hydrochlorid,
[2-(3'-Isopropoxybiphenyl-2-yl)ethyl]dimethylamin und das entsprechende Hydrochlorid,
[2-(4'-Chlor-3'-methoxybiphenyl-2-yl)ethyl]dimethylamin und das entsprechende Hydrochlorid,
4-Chlor-2'-(2-dimethylaminoethyl)biphenyl-3-ol und das entsprechende Hydrochlorid,
Dimethyl-(3'-nitrobiphenyl-2-ylmethyl)amin und das entsprechende Hydrochlorid,
4-Amino-2'-dimethylaminomethylbiphenyl-3-ol und das entsprechende Dihydrochlorid,
(3',5'-Difluorbiphenyl-2-ylmethyl)dimethylamin und das entsprechende Hydrochlorid,
(2',5'-Dimethoxybiphenyl-2-ylmethyl)dimethylamin und das entsprechende Hydrochlorid,
2'-Dimethylaminomethyl-5-trifluormethoxybiphenyl-2-ylamin und das entsprechende Dihydrochlorid,
N-(2'-Dimethylaminomethyl-5-trifluormethoxybiphenyl-2-yl)acetamid und das entsprechende Hydrochlorid sowie
3,5-Dichlor-2'-dimethylaminomethyl-biphenyl-4-ylamin und das entsprechende Hydrochlorid.

## Claims

**1.** Substituted 2-aminoalkylbiphenyl derivatives of the general formula I

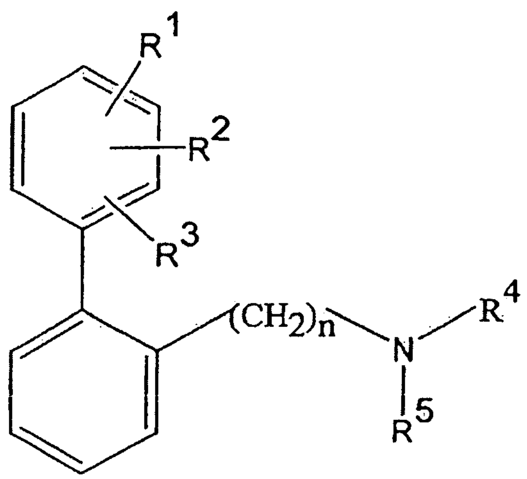

I

wherein

n is 1 or 2,

the radical $R^1$ represents CN, $NO_2$, $SO_2CH_3$, $SO_2CF_3$, O-aryl, O-$C_{1-6}$-alkylene-aryl, $NR^6R^7$, an aryl, an acetyl or an acetamidyl radical, or represents an aryl radical bonded via a $C_{1-6}$-alkylene group,
the radical $R^2$ represents H, F, Cl, Br, CN, $NO_2$ CHO, $SO_2CH_3$, $SO_2CF_3$, $OR^6$, $NR^6R^7$, a $C_{1-6}$-alkyl, an aryl, an acetyl, an acetamidyl or a benzoyl radical or represents an aryl radical bonded via a $C_{1-6}$-alkylene group,
or $R^1$ and $R^2$ together in each case denote the group $OCH_2CH_2O$, CH=CHO, CH=C ($CH_3$) O or CH=CHNH,
the radical $R^3$ represents H, F, Cl, Br, CN, $NO_2$, CHO, $SO_2CH_3$, $SO_2CF_3$, $OR^6$, $NR^6R^7$, a $C_{1-6}$-alkyl, an aryl, an acetyl, an acetamidyl or a benzoyl radical or represents an aryl radical bonded via a $C_{1-6}$-alkylene group,
the radicals $R^4$, $R^5$, which are identical or different, represent H, or represent a $C_{1-6}$-alkyl radical,
the radicals $R^6$, $R^7$, which are identical or different, represent H, a $C_{1-6}$-alkyl or an aryl radical, or represent an aryl radical bonded via a $C_{1-6}$-alkylene group,
wherein
the alkyl radicals in each case can also be substituted at least once in an identical or different manner by halogen and/or a hydroxyl group, and
the aryl radicals in each case can also denote phenyls or naphthyl radicals which are at least monosubstituted by an OH, a halogen, a $CF_3$, a $C_{1-6}$-alkyl, a $C_{1-6}$-alkoxy, a $C_{1-7}$-cycloalkoxy, a $C_{3-7}$-cycloalkyl, a $C_{2-6}$-alkylene or a phenyl radical,
in the form of their bases and/or salts of physiologically tolerated acids, the compounds
4-chloro-2'-dimethylaminomethylbiphenyl-2-carbonitrile and
4-(2'-N,N-dimethylaminomethylphenyl)-2-fluoroaniline
being excluded.

**2.** Substituted 2-aminoalkylbiphenyl derivatives according to claim 1, **characterized in that** the radicals $R^2$ and/or $R^3$ represent a $C_{1-3}$-alkyl radical and the other substituents and n have the meaning according to the general formula I.

**3.** Substituted 2-aminoalkylbiphenyl derivatives according to claim 1 or 2, **characterized in that** the radicals $R^1$, $R^2$ and/or $R^3$ represent an aryl radical bonded via a $C_{1-3}$-alkylene group and the other substituents and n have the meaning according to the general formula I.

**4.** Substituted 2-aminoalkylbiphenyl derivatives according to one or more of claims 1 to 3, **characterized in that** the radicals $R^4$ and/or $R^5$ represent a $C_{1-3}$-alkyl radical and the other substituents and n have the meaning according to the general formula I.

**5.** Substituted 2-aminoalkylbiphenyl derivatives according to one or more of claims 1 to 4, **characterized in that** the radicals $R^6$ and/or $R^7$ represent a $C_{1-3}$-alkyl radical and the other substituents and n have the meaning according to the general formula I.

**6.** Substituted 2-aminoalkylbiphenyl derivatives according to one or more of claims 1 to 5, **characterized in that** the radicals $R^6$ and/or $R^7$ represent an aryl radical bonded via a $C_{1-3}$-alkylene group and the other substituents and n have the meaning according to the general formula I.

**7.** Substituted 2-aminoalkylbiphenyl derivatives according to claim 1,
dimethyl-[2-(2-methylbenzofuran-4-yl)benzyl]amine and the corresponding hydrochloride
dimethyl-(4'-nitro-3'-trifluoromethylbiphenyl-2-ylmethyl)-amine and the corresponding hydrochloride
*N*-(2'-dimethylaminomethyl-3-trifluoromethoxybiphenyl-4-yl)acetamide and the corresponding hydrochloride
[2-(1*H*-indol-5-yl)benzyl]dimethylamine and the corresponding hydrochloride
(4'-methanesulfonylbiphenyl-2-ylmethyl)dimethylamine and the corresponding hydrochloride
1-[2'-(2-dimethylaminoethyl)biphenyl-3-yl]ethanone and the corresponding hydrochloride
dimethyl-(3'-nitrobiphenyl-2-ylmethyl)amine and the corresponding hydrochloride
4-amino-2'-dimethylaminomethylbiphenyl-3-ol and the corresponding dihydrochloride
2'-dimethylaminomethyl-5-trifluoromethoxybiphenyl-2-ylamine and the corresponding dihydrochloride
N-(2'-dimethylaminomethyl-5-trifluoromethoxybiphenyl-2-yl)acetamide and the corresponding hydrochloride and
3,5-dichloro-2'-dimethylaminomethyl-biphenyl-4-ylamine and the corresponding hydrochloride.

**8.** Process for the preparation of substituted 2-aminoalkyl-biphenyl derivatives of the general formula I according to one or more of claims 1 to 7, **characterized in that** the compounds of the general formula II

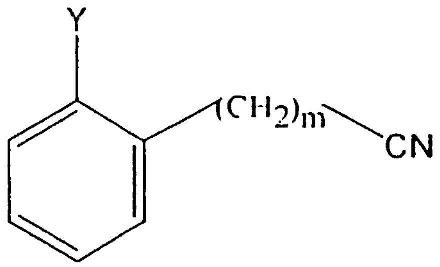

II

wherein Y denotes Cl, Br or I and m denotes 0 or 1, are reduced in solution with a reducing agent to give compounds of the general formula III, wherein n denotes 1 or 2

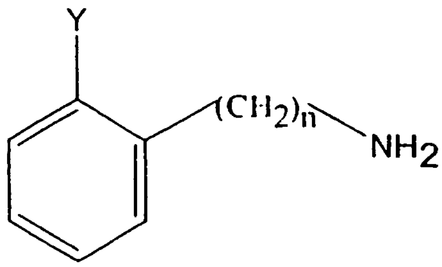

III

and these are purified and isolated by conventional methods,
the compounds of the general formula III are reacted with aliphatic $C_{1-6}$-aldehydes in the presence of a reducing agent to give compounds of the general formula IV

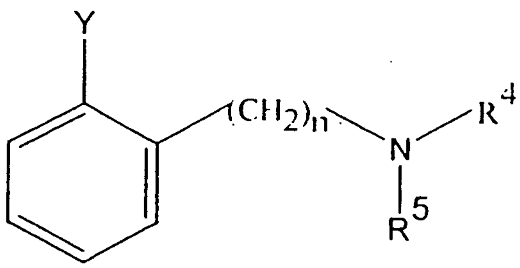

IV

wherein $R^4$ and $R^5$ have the meaning according to the general formula I according to claim 1, and these are purified and isolated by conventional methods,
the compounds of the general formula IV are converted by halogen-metal exchange and subsequent reaction with a boric acid ester at a temperature of ≤ 0°C to give compounds of the general formula V, wherein R denotes a $C_{1-6}$-alkyl radical

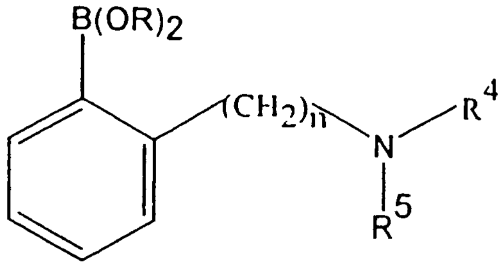

V

and these are isolated and purified by conventional methods or employed in the following reaction step without further purification,
the compounds of the general formula V are reacted with aqueous acids to give compounds of the general formula VI

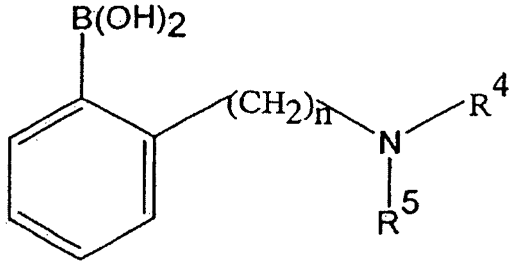

VI

and these are purified and isolated by conventional methods,
the compounds of the general formula V or VI are reacted in a transition metal-catalysed reaction in an aliphatic ether, a hydrocarbon, an alcohol, a chlorinated hydrocarbon, water or mixtures of these solvents at temperatures between 20 and 150°C with compounds of the general formula VII

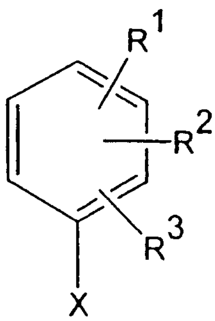

VII,

wherein X denotes Cl, Br, I or $OSO_2CpF_{(2p+1)}$ and the radicals $R^1$ to $R^3$ have the meaning according to the general formula I according to claim 1, to give compounds of the general formula I
and these are purified and isolated by conventional methods, or
the compounds of the general formula VIII or IX

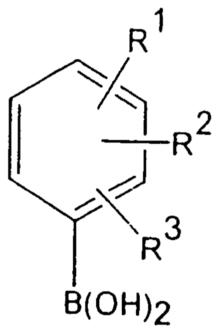

VIII

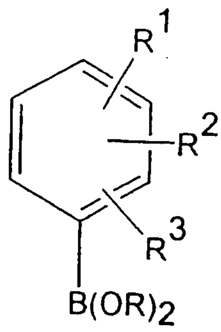

IX,

wherein $R^1$ to $R^3$ have the meaning according to the general formula I according to claim 1, are reacted in a transition metal-catalysed reaction in an aliphatic ether, a hydrocarbon, an alcohol, a chlorinated hydrocarbon, water or mixtures of these solvents at temperatures between 20 and 150°C with compounds of the general formula III or IV to give compounds of the general formula I, and these are purified and isolated by conventional methods.

**9.** Process according to claim 8, **characterized in that** the compounds of the general formula II are reduced with lithium aluminium hydride and/or diisobutylaluminium hydride.

**10.** Process according to claim 8 or 9, **characterized in that** the compounds of the general formula III are reacted with aliphatic $C_{1-6}$-aldehydes in the presence of formic acid and/or sodium borohydride.

**11.** Process according to one or more of claims 8 to 10, **characterized in that** the halogen-metal exchange is carried out with magnesium and/or butyllithium.

**12.** Process according to one or more of claims 8 to 11, **characterized in that** the boric acid ester is a trialkyl borate, preferably trimethyl borate.

**13.** Process according to one or more of claims 8 to 12, **characterized in that** the compounds of the general formula V are reacted with hydrochloric acid to give compounds of the general formula VI.

**14.** Process according to one or more of claims 8 to 13, **characterized in that** the compounds of the general formula V or VI are reacted in a reaction catalysed by palladium(0) compounds and/or by palladium(II) salts, preferably by tetrakis(triphenylphosphine)palladium, bis(dibenzylideneacetone)palladium, elemental palladium on active charcoal, palladium (II) chloride and/or palladium (II) acetate.

**15.** Process according to one or more of claims 8 to 13, **characterized in that** the compounds of the general formula VIII or IX are reacted in a reaction catalysed by palladium(O) compounds and/or by palladium(II) salts, preferably by tetrakis(triphenylphosphine) palladium, bis(dibenzylideneacetone)palladium, elemental palladium on active charcoal, palladium(II) chloride and/or palladium(II) acetate.

**16.** Process according to one or more of claims 8 to 15, **characterized in that** the transition metal-catalysed reaction is carried out in 1,4-dioxane, tetrahydrofuran, toluene, hexane, ethanol, isopropanol, chloroform, methylene chloride, water or mixtures of these solvents.

**17.** Medicaments comprising, as the pharmaceutical active compound, at least one substituted 2-aminoalkylbiphenyl derivative of the general formula I

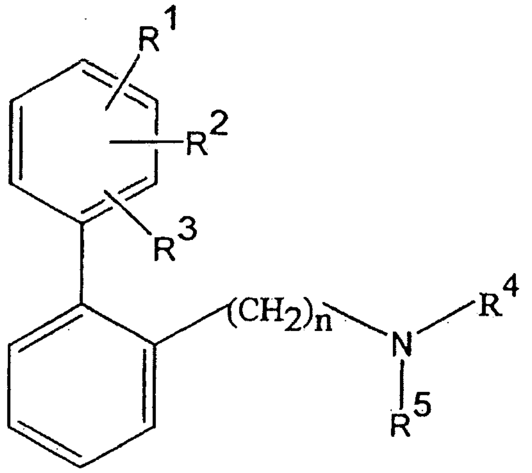

I

wherein

n is 1 or 2,
the radicals $R^1$, $R^2$, $R^3$, which are identical or different, represent H, F, Cl, Br, CN, $NO_2$, CHO, $SO_2CH_3$, $SO_2CF_3$, $OR^6$, $NR^6R^7$, a $C_{1-6}$-alkyl, an aryl, an acetyl, an acetamidyl or a benzoyl radical or represent an aryl radical bonded via a $C_{1-6}$-alkylene group,
or $R^1$ and $R^2$ together in each case denote the group $OCH_2O$, $OCH_2CH_2O$, CH=CHO, $CH=C(CH_3)O$ or CH=CHNH,
the radicals $R^4$, $R^5$, which are identical or different, represent H, or represent a $C_{1-6}$-alkyl radical,
the radicals $R^6$, $R^7$, which are identical or different, represent H, a $C_{1-6}$-alkyl or an aryl radical, or represent an aryl radical bonded via a $C_{1-6}$-alkylene group,
wherein the alkyl radicals in each case can also be substituted at least once in an identical or different manner by halogen and/or a hydroxyl group, and
the aryl radicals in each case can also denote phenyls or naphthyl radicals which are at least monosubstituted by an OH, a halogen, a $CF_3$, a $C_{1-6}$alkyl, a $C_{1-6}$-alkoxy, a $C_{1-7}$-cycloalkoxy, a $C_{3-7}$cycloalkyl, a $C_{2-6}$-alkylene or a phenyl radical,
in the form of its base and/or of a salt of a physiologically tolerated acid and optionally further active compounds and/or auxiliary substances.

**18.** Medicaments according to claim 17, comprising, as the pharmaceutical active compound, at least one substituted 2-aminoalkylbiphenyl derivative chosen from the group which includes the following compounds:

(3'-methoxybiphenyl-2-ylmethyl)dimethylamine and the corresponding hydrochloride,
(4'-chlorobiphenyl-2-ylmethyl)dimethylamine and the corresponding hydrochloride,
2'-dimethylaminomethylbiphenyl-3-ol and the corresponding hydrochloride,
(2'-methoxybiphenyl-2-ylmethyl)dimethylamine and the corresponding hydrochloride,
(3'-chlorobiphenyl-2-ylmethyl)dimethylamine and the corresponding hydrochloride,
(2'-fluorobiphenyl-2-ylmethyl)dimethylamine and the corresponding hydrochloride,
(3'-fluorobiphenyl-2-ylmethyl)dimethylamine and the corresponding hydrochloride,
(4'-fluorobiphenyl-2-ylmethyl)dimethylamine and the corresponding hydrochloride,
(3'-chloro-4'-fluorobiphenyl-2-ylmethyl)dimethylamine and the corresponding hydrochloride,
(3'-methoxybiphenyl-2-ylethyl)dimethylamine and the corresponding hydrochloride,
dimethyl-[2-(2-methylbenzofuran-4-yl)benzyl]amine and the corresponding hydrochloride,
2'-dimethylaminomethylbiphenyl-2-carbaldehyde hydrochloride,
(3'-difluoromethylbiphenyl-2-ylmethyl)dimethylamine and the corresponding hydrochloride,
2'-dimethylaminomethylbiphenyl-3-carbaldehyde and the corresponding hydrochloride,
biphenyl-2-ylmethyldimethylamine hydrochloride,
(3',4'-dichlorobiphenyl-2-ylmethyl)dimethylamine and the corresponding hydrochloride,
(3',5'-dichlorobiphenyl-2-ylmethyl)dimethylamine and the corresponding hydrochloride,

dimethyl-(4'-nitro-3'-trifluoromethylbiphenyl-2-ylmethyl)-amine and the corresponding hydrochloride,
(3',4'-difluorobiphenyl-2-ylmethyl)dimethylamine and the corresponding hydrochloride,
(4'-fluoro-3'-trifluoromethylbiphenyl-2-ylmethyl)dimethyl-amine and the corresponding hydrochloride,
(4'-chloro-3'-methoxybiphenyl-2-ylmethyl)dimethylamine and the corresponding hydrochloride,
*N*-(2'-dimethylaminomethyl-3-trifluoromethoxybiphenyl-4-yl)acetamide and the corresponding hydrochloride,
(3'-isopropoxybiphenyl-2-ylmethyl)dimethylamine and the corresponding hydrochloride,
2'-(2-dimethylaminoethyl)biphenyl-3-ol and the corresponding hydrochloride,
4-chloro-2'-dimethylaminomethylbiphenyl-3-ol and the corresponding hydrochloride,
[2-(1*H*-indol-5-yl)benzyl]dimethylamine and the corresponding hydrochloride,
(4'-methanesulfonylbiphenyl-2-ylmethyl)dimethylamine and the corresponding hydrochloride,
(2',4'-dichlorobiphenyl-2-ylmethyl)dimethylamine and the corresponding hydrochloride,
(2',3'-difluorobiphenyl-2-ylmethyl)dimethylamine and the corresponding hydrochloride,
(2',5'-difluorobiphenyl-2-ylmethyl)dimethylamine and the corresponding hydrochloride,
(2-benzo[1.3]dioxol-5-ylbenzyl)dimethylamine and the corresponding hydrochloride,
1-[2'-(2-dimethylaminoethyl)biphenyl-3-yl]ethanone and the corresponding hydrochloride,
[2-(3',4'-dimethoxybiphenyl-2-yl)ethyl]dimethylamine and the corresponding hydrochloride,
[2-(3'-isopropoxybiphenyl-2-yl)ethyl]dimethylamine and the corresponding hydrochloride,
[2-(4'-chloro-3'-methoxybiphenyl-2-yl)ethyl]dimethylamine and the corresponding hydrochloride,
4-chloro-2'-(2-dimethylaminoethyl)biphenyl-3-ol and the corresponding hydrochloride
dimethyl-(3'-nitrobiphenyl-2-ylmethyl)amine and the corresponding hydrochloride
4-amino-2'-dimethylaminomethylbiphenyl-3-ol and the corresponding dihydrochloride
(3',5'-difluorobiphenyl-2-ylmethyl)dimethylamine and the corresponding hydrochloride
(2',5'-dimethoxybiphenyl-2-ylmethyl)dimethylamine and the corresponding hydrochloride
2'-dimethylaminomethyl-5-trifluoromethoxybiphenyl-2-ylamine and the corresponding dihydrochloride
N-(2'-dimethylaminomethyl-5-trifluoromethoxybiphenyl-2-yl)acetamide and the corresponding hydrochloride and
3,5-dichloro-2'-dimethylaminomethyl-biphenyl-4-ylamine and the corresponding hydrochloride.

**19.** Medicaments according to claim 17 or 18 for treatment/control for/of pain, inflammatory and allergic reactions, depression, drug and alcohol abuse, gastritis, diarrhoea, urinary incontinence, cardiovascular diseases, respiratory tract diseases, coughing, mental illnesses and/or epilepsy.

**20.** Use of at least one substituted 2-aminoalkyl-biphenyl derivative of the general formula I according to claim 17 in the form of its base and/or of a salt of a physiologically tolerated acid for the preparation of a medicament for controlling pain.

**21.** Use of at least one substituted 2-aminoalkyl-biphenyl derivative of the general formula I according to claim 17 in the form of its bases and/or of a salt of physiologically tolerated acids for the preparation of a medicament for treatment of inflammatory reactions.

**22.** Use of at least one substituted 2-aminoalkyl-biphenyl derivative of the general formula I according to claim 17 in the form of its base and/or of a salt of a physiologically tolerated acid for the preparation of a medicament for treatment of allergic reactions.

**23.** Use of at least one substituted 2-aminoalkyl-biphenyl derivative of the general formula I according to claim 17 in the form of its base and/or of a salt of a physiologically tolerated acid for the preparation of a medicament for treatment of depression.

**24.** Use of at least one substituted 2-aminoalkyl-biphenyl derivative of the general formula I according to claim 17 in the form of its bases and/or of a salt of physiologically tolerated acids for the preparation of a medicament for treatment of drug and/or alcohol abuse.

**25.** Use of at least one substituted 2-aminoalkyl-biphenyl derivative of the general formula I according to claim 17 in the form of its base and/or of a salt of a physiologically tolerated acid for the preparation of a medicament for treatment of gastritis.

**26.** Use of at least one substituted 2-aminoalkyl-biphenyl derivative of the general formula I according to claim 17 in the form of its base and/or of a salt of a physiologically tolerated acid for the preparation of a medicament for treatment

of diarrhoea.

**27.** Use of at least one substituted 2-aminoalkyl-biphenyl derivative of the general formula I according to claim 17 in the form of its base and/or of a salt of a physiologically tolerated acid for the preparation of a medicament for treatment of urinary incontinence.

**28.** Use of at least one substituted 2-aminoalkyl-biphenyl derivative of the general formula I according to claim 17 in the form of its base and/or of a salt of a physiologically tolerated acid for the preparation of a medicament for treatment of cardiovascular diseases.

**29.** Use of at least one substituted 2-aminoalkyl-biphenyl derivative of the general formula I according to claim 17 in the form of its base and/or of a salt of a physiologically tolerated acid for the preparation of a medicament for treatment of respiratory tract diseases.

**30.** Use of at least one substituted 2-aminoalkyl-biphenyl derivative of the general formula I according to claim 17 in the form of its base and/or of a salt of a physiologically tolerated acid for the preparation of a medicament for treatment of coughing.

**31.** Use of at least one substituted 2-aminoalkyl-biphenyl derivative of the general formula I according to claim 17 in the form of its base and/or of a salt of a physiologically tolerated acid for the preparation of a medicament for treatment of mental illnesses.

**32.** Use of at least one substituted 2-aminoalkyl-biphenyl derivative of the general formula I according to claim 17 in the form of its base and/or of a salt of a physiologically tolerated acid for the preparation of a medicament for treatment of epilepsy.

**33.** Use according to one of claims 20-32, **characterized in that** the substituted 2-aminoalkyl-biphenyl derivative is chosen from the group which includes the following compounds:

(3'-methoxybiphenyl-2-ylmethyl)dimethylamine and the corresponding hydrochloride,
(4'-chlorobiphenyl-2-ylmethyl)dimethylamine and the corresponding hydrochloride,
2'-dimethylaminomethylbiphenyl-3-ol and the corresponding hydrochloride,
(2'-methoxybiphenyl-2-ylmethyl)dimethylamine and the corresponding hydrochloride,
(3'-chlorobiphenyl-2-ylmethyl)dimethylamine and the corresponding hydrochloride,
(2'-fluorobiphenyl-2-ylmethyl)dimethylamine and the corresponding hydrochloride,
(3'-fluorobiphenyl-2-ylmethyl)dimethylamine and the corresponding hydrochloride,
(4'-fluorobiphenyl-2-ylmethyl)dimethylamine and the corresponding hydrochloride,
(3'-chloro-4'-fluorobiphenyl-2-ylmethyl)dimethylamine and the corresponding hydrochloride,
(3'-methoxybiphenyl-2-ylethyl)dimethylamine and the corresponding hydrochloride,
dimethyl-[2-(2-methylbenzofuran-4-yl)benzyl]amine and the corresponding hydrochloride,
2'-dimethylaminomethylbiphenyl-2-carbaldehyde hydrochloride,
(3'-difluoromethylbiphenyl-2-ylmethyl)dimethylamine and the corresponding hydrochloride,
2'-dimethylaminomethylbiphenyl-3-carbaldehyde and the corresponding hydrochloride,
biphenyl-2-ylmethyldimethylamine hydrochloride,
(3',4'-dichlorobiphenyl-2-ylmethyl)dimethylamine and the corresponding hydrochloride,
(3',5'-dichlorobiphenyl-2-ylmethyl)dimethylamine and the corresponding hydrochloride,
dimethyl-(4'-nitro-3'-trifluoromethylbiphenyl-2-ylmethyl)-amine and the corresponding hydrochloride,
(3',4'-difluorobiphenyl-2-ylmethyl)dimethylamine and the corresponding hydrochloride,
(4'-fluoro-3'-trifluoromethylbiphenyl-2-ylmethyl)dimethyl-amine and the corresponding hydrochloride,
(4'-chloro-3'-methoxybiphenyl-2-ylmethyl)dimethylamine and the corresponding hydrochloride,
*N*-(2'-dimethylaminomethyl-3-trifluoromethoxybiphenyl-4-yl)acetamide and the corresponding hydrochloride,
(3'-isopropoxybiphenyl-2-ylmethyl)dimethylamine and the corresponding hydrochloride,
2'-(2-dimethylaminoethyl)biphenyl-3-ol and the corresponding hydrochloride,
4-chloro-2'-dimethylaminomethylbiphenyl-3-ol and the corresponding hydrochloride,
[2-(1*H*-indol-5-yl)benzyl]dimethylamine and the corresponding hydrochloride,
(4'-methanesulfonylbiphenyl-2-ylmethyl)dimethylamine and the corresponding hydrochloride,
(2',4'-dichlorobiphenyl-2-ylmethyl)dimethylamine and the corresponding hydrochloride,
(2',3'-difluorobiphenyl-2-ylmethyl)dimethylamine and the corresponding hydrochloride,

(2',5'-difluorobiphenyl-2-ylmethyl)dimethylamine and the corresponding hydrochloride,
(2-benzo[1.3]dioxol-5-ylbenzyl)dimethylamine and the corresponding hydrochloride,
1-[2'-(2-dimethylaminoethyl)biphenyl-3-yl]ethanone and the corresponding hydrochloride,
[2-(3',4'-dimethoxybiphenyl-2-yl)ethyl]dimethylamine and the corresponding hydrochloride,
[2-(3'-isopropoxybiphenyl-2-yl)ethyl]dimethylamine and the corresponding hydrochloride,
[2-(4'-chloro-3'-methoxybiphenyl-2-yl)ethyl]dimethylamine and the corresponding hydrochloride,
4-chloro-2'-(2-dimethylaminoethyl)biphenyl-3-ol and the corresponding hydrochloride
dimethyl-(3'-nitrobiphenyl-2-ylmethyl)amine and the corresponding hydrochloride
4-amino-2'-dimethylaminomethylbiphenyl-3-ol and the corresponding dihydrochloride
(3',5'-difluorobiphenyl-2-ylmethyl)dimethylamine and the corresponding hydrochloride
(2',5'-dimethoxybiphenyl-2-ylmethyl)dimethylamine and the corresponding hydrochloride
2'-dimethylaminomethyl-5-trifluoromethoxybiphenyl-2-ylamine and the corresponding dihydrochloride
N-(2'-dimethylaminomethyl-5-trifluoromethoxybiphenyl-2-yl)acetamide and the corresponding hydrochloride and
3,5-dichloro-2'-dimethylaminomethyl-biphenyl-4-ylamine and the corresponding hydrochloride.

## Revendications

1. Dérivés substitués de 2-aminoalkylbiphényles de formule générale I

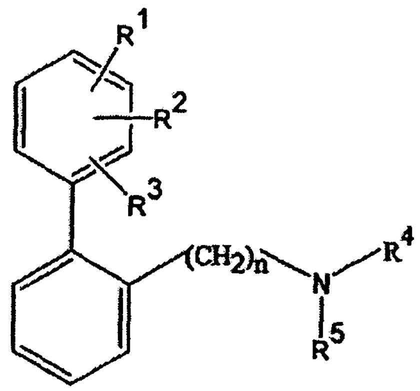

I

dans laquelle

n a la valeur 1 ou 2,
le reste $R^1$ représente un groupe CN, $NO_2$, $SO_2CH_3$, $SO_2CF_3$, O-aryle, O-(alkylène en $C_1$ à $C_6$)-aryle, $NR^6R^7$, un reste aryle, un reste acétyle, un reste acétamidyle ou un reste aryle lié par l'intermédiaire d'un groupe alkylène en $C_1$ à $C_6$,
le reste $R^2$ représente H, F, Cl, Br, un groupe CN, $NO_2$ CHO, $SO_2CH_3$, $SO_2CF_3$, $OR^6$, $NR^6R^7$, un reste alkyle en $C_1$ à $C_6$, un reste aryle, un reste acétyle, un reste acétamidyle, un reste benzoyle ou un reste aryle lié par l'intermédiaire d'un groupe alkylène en $C_1$ à $C_6$,
ou bien $R^1$ et $R^2$ forment ensemble respectivement le groupe $OCH_2CH_2O$, CH=CHO, $CH{=}C(CH_3)O$ ou CH=CH-NH,
le reste $R^3$ représente H, F, Cl, Br, un groupe CN, $NO_2$, CHO, $SO_2CH_3$, $SO_2CF_3$, $OR^6$, $NR^6R^7$, un reste alkyle en $C_1$ à $C_6$, un reste aryle, un reste acétyle, un reste acétamidyle, un reste benzoyle ou un reste aryle lié par l'intermédiaire d'un groupe alkylène en $C_1$ à $C_6$,
les restes $R^4$, $R^5$, identiques ou différents, représentent H ou un reste alkyle en $C_1$ à $C_6$,
les restes $R^6$, $R^7$, identiques ou différents, représentent H, un reste alkyle en $C_1$ à $C_6$, un reste aryle ou un reste aryle lié par l'intermédiaire d'un groupe alkylène en $C_1$ à $C_6$,
les restes alkyle pouvant également dans chaque cas être substitués au moins une fois, de façon identique ou différente, avec un halogène et/ou un groupe hydroxy, et

les restes aryle pouvant également représenter des restes phényle ou naphtyle substitués dans chaque cas au moins une fois avec un groupe OH, un halogène, un reste $CF_3$, un reste alkyle en $C_1$ à $C_6$, un reste alkoxy en $C_1$ à $C_6$, un reste cycloalkoxy en $C_1$ à $C_7$, un reste cycloalkyle en $C_3$ à $C_7$, un reste alkylène en $C_2$ à $C_6$ ou un reste phényle,
sous forme de leurs bases et/ou de sels d'acides acceptables du point de vue physiologique, les composés 4-chloro-2'-diméthylaminométhylbiphényl-2-carbonitrile et 4-(2'-N,N-diméthylaminométhylphényl)-2-fluoraniline étant exclus.

2. Dérivés substitués de 2-aminoalkylbiphényles suivant la revendication 1, **caractérisés en ce que** les restes $R^2$ et/ou $R^3$ représentent un reste alkyle en $C_1$ à $C_3$ et les autres substituants ainsi que n ont la définition selon la formule générale I.

3. Dérivés substitués de 2-aminoalkylbiphényles selon la revendication 1 ou 2, **caractérisés en ce que** les restes $R^1$, $R^2$ et/ou $R^3$ représentent un reste aryle lié par l'intermédiaire d'un groupe alkylène en $C_1$ à $C_3$ et les autres substituants ainsi que n ont la définition selon la formule générale I.

4. Dérivés substitués de 2-aminoalkylbiphényles selon l'une ou plusieurs des revendications 1 à 3, **caractérisés en ce que** les restes $R^4$ et/ou $R^5$ représentent un reste alkyle en $C_1$ à $C_3$ et les autres substituants ainsi que n ont la définition selon la formule générale I.

5. Dérivés substitués de 2-aminoalkylbiphényles selon l'une ou plusieurs des revendications 1 à 4, **caractérisés en ce que** les restes $R^6$ et/ou $R^7$ représentent un reste alkyle en $C_1$ à $C_3$ et les autres substituants ainsi que n ont la définition selon la formule générale I.

6. Dérivés substitués de 2-aminoalkylbiphényles suivant l'une ou plusieurs des revendications 1 à 5, **caractérisés en ce que** les restes $R^6$ et/ou $R^7$ représentent un reste aryle lié par l'intermédiaire d'un groupe alkylène en $C_1$ à $C_3$ et les autres substituants ainsi que n ont la définition selon la formule générale I.

7. Dérivés substitués de 2-aminoalkylbiphényles suivant la revendication 1 :

diméthyl-[2-(2-méthylbenzofuranne-4-yl)benzyl]-amine et le chlorhydrate correspondant
diméthyl-(4'-nitro-3'-trifluorométhylbiphényl-2-yl-méthyl)-amine et le chlorhydrate correspondant
*N*-(2'-diméthylaminométhyl-3-trifluorométhoxybiphényl-4-yl)-acétamide et le chlorhydrate correspondant
[2-(1*H*-indole-5-yl)benzyl]-diméthylamine et le chlorhydrate correspondant
(4'-méthanesulfonylbiphényl-2-ylméthyl)-diméthylamine et le chlorhydrate correspondant
1-[2'-(2-diméthylaminoéthyl)biphényl-3-yl]-éthanone et le chlorhydrate correspondant
diméthyl-(3'-nitrobiphényl-2-ylméthyl)-amine et le chlorhydrate correspondant
4-amino-2'-diméthylaminométhylbiphényl-3-ol et le dichlorhydrate correspondant
2'-diméthylaminométhyl-5-trifluorométhoxybiphényl-2-ylamine et le dichlorhydrate correspondant
N-(2'-diméthylaminométhyl-5-trifluorométhoxybiphényl-2-yl)-acétamide et le chlorhydrate correspondant
3,5-dichloro-2'-diméthylaminométhyl-biphényl-4-ylamine et le chlorhydrate correspondant.

8. Procédé de production de dérivés substitués de 2-aminoalkylbiphényles de formule générale I selon l'une ou plusieurs des revendications 1 à 7, **caractérisé en ce qu'**on réduit les composés de formule générale II

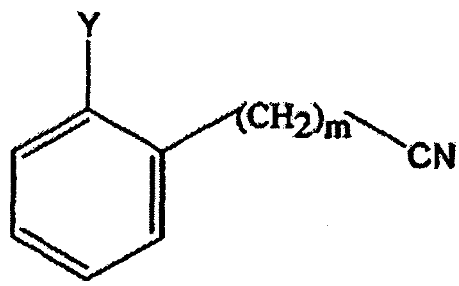

II,

dans laquelle Y représente Cl, Br ou I et m a la valeur 0 ou 1, en solution, avec un agent réducteur, en composés de formule générale III dans laquelle n a la valeur 1 ou 2

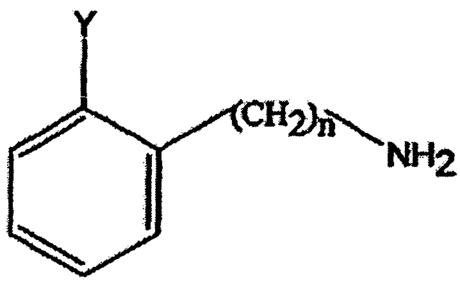

III

qu'on purifie et isole selon des modes opératoires usuels,
on fait réagir les composés de formule générale III en présence d'un agent réducteur avec des aldéhydes aliphatiques en $C_1$ à $C_6$ pour obtenir des composés de formule générale IV

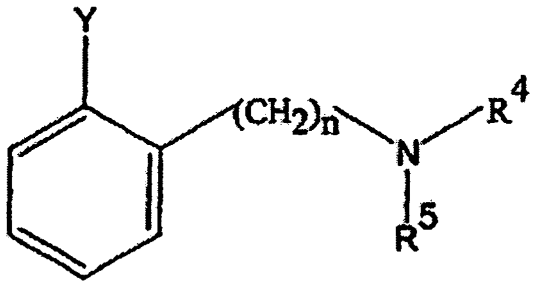

IV,

dans laquelle $R^4$ et $R^5$ ont la définition de la formule générale I selon la revendication 1, et on purifie et isole ces composés selon des modes opératoires usuels,
on fait réagir les composés de formule générale IV par échange halogène-métal suivi d'une réaction avec un ester d'acide borique à une température inférieure ou égale à 0°C pour obtenir des composés de formule générale V dans laquelle R est un reste alkyle en $C_1$ à $C_6$

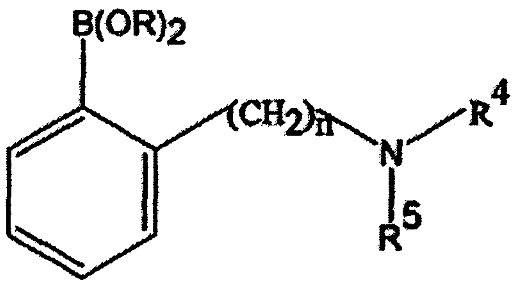

V,

et on isole et purifie ces composés selon des modes opératoires usuels ou on les utilise sans autre purification dans l'étape réactionnelle suivante,
on fait réagir les composés de formule générale V avec des acides aqueux pour former des composés de formule générale VI

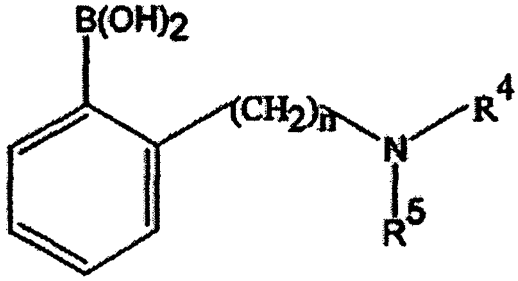

VI

qu'on purifie et isole par des modes opératoires usuels,
on fait réagir les composés de formule générale V ou VI dans une réaction catalysée par un métal de transition dans un éther aliphatique, un hydrocarbure, un alcool, un hydrocarbure chloré, l'eau ou des mélanges de ces solvants à des températures comprises entre 20 et 150°C, avec des composés de formule générale VII

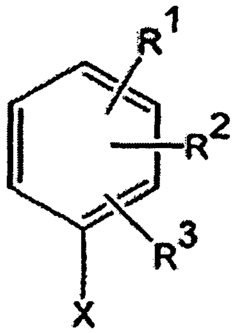

VII,

dans laquelle X représente Cl, Br, I ou un groupe $OSO_2C_pF_{(2p+1)}$ et les restes $R^1$ à $R^3$ sont définis conformément à la formule générale I selon la revendication 1, pour obtenir des composés de formule générale I
qu'on purifie et isole selon des modes opératoires usuels, ou bien
on fait réagir les composés de formule générale VIII ou IX

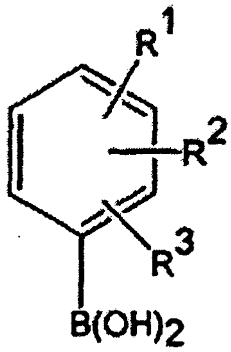

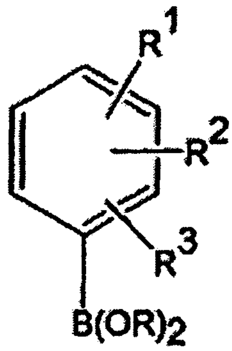

VIII IX,

dons laquelle $R^1$ à $R^3$ sont définis conformément à la formule générale I selon la revendication 1, dans une réaction catalysée par un métal de transition dans un éther aliphatique, un hydrocarbure, un alcool, un hydrocarbure chloré,

l'eau ou des mélanges de ces solvants à des températures comprises entre 20 et 150°C, avec des composés de formule générale III ou IV, pour obtenir des composés de formule générale I qu'on purifie et isole selon des modes opératoires usuels.

**9.** Procédé suivant la revendication 8, **caractérisé en ce qu'**on réduit les composés de formule générale II avec l'hydrure de lithium et d'aluminium et/ou l'hydrure de diisobutylaluminium.

**10.** Procédé suivant la revendication 8 ou 9, **caractérisé en ce qu'**on fait réagir les composés de formule générale III avec des aldéhydes aliphatiques en $C_1$ à $C_6$ en présence d'acide formique et/ou de borohydrure de sodium.

**11.** Procédé suivant l'une ou plusieurs des revendications 8 à 10, **caractérisé en ce que** l'échange halogène-métal est conduit avec le magnésium et/ou le butyllithium.

**12.** Procédé suivant l'une ou plusieurs des revendications 8 à 11, **caractérisé en ce que** l'ester d'acide borique est un borate de trialkyle, avantageusement le borate de triméthyle.

**13.** Procédé suivant l'une ou plusieurs des revendications 8 à 12, **caractérisé en ce qu'**on fait réagir les composés de formule générale V avec l'acide chlorhydrique pour obtenir des composés de formule générale VI.

**14.** Procédé suivant l'une ou plusieurs des revendications 8 à 13, **caractérisé en ce qu'**on fait réagir les composés de formule générale V ou VI dans une réaction catalysée par des composés de palladium (0) et/ou par des sels de palladium (II), avantageusement par le tétrakis-(triphénylphosphine)-palladium, le bis-(dibenzylidène-acétone)-palladium, le palladium élémentaire sur du charbon actif, le chlorure de palladium (II) et/ou l'acétate de palladium (II).

**15.** Procédé suivant l'une ou plusieurs des revendications 8 à 13, **caractérisé en ce qu'**on fait réagir les composés de formule générale VIII ou IX dans une réaction catalysée par des composés de palladium(0) ou par des sels de palladium (II), avantageusement par le tétrakis-(triphénylphosphine)-palladium, le bis-(dibenzylidèneacétone)-palladium, le palladium élémentaire sur du charbon actif, le chlorure de palladium(II) et/ou l'acétate de palladium(II).

**16.** Procédé suivant l'une ou plusieurs des revendications 8 à 15, **caractérisé en ce que** la réaction catalysée par un métal de transition est conduite dans le 1,4-dioxanne, le tétrahydrofuranne, le toluène, l'hexane, l'éthanol, l'isopropanol, le chloroforme, le dichlorométhane, l'eau ou des mélanges de ces solvants.

**17.** Médicament contenant, comme substance pharmaceutique, au moins un dérivé substitué de 2-aminoalkylbiphényle de formule générale I

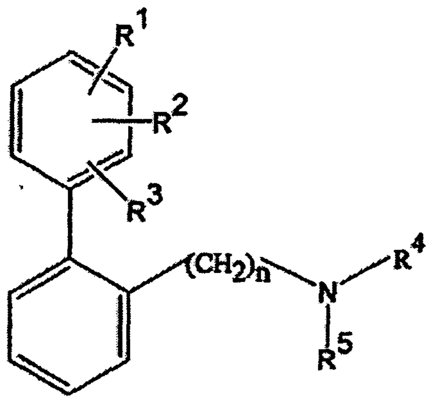

I

dans laquelle

n a la valeur 1 ou 2,
les restes $R^1$, $R^2$, $R^3$ identiques ou différents, représentent H, F, Cl, Br, CN, $NO_2$, CHO, $SO_2CH_3$, $SO_2CF_3$, $OR^6$, $NR^6R^7$, un reste alkyle en $C_1$ à $C_6$, un reste aryle, un reste acétyle, un reste acétamidyle, un reste benzoyle

ou un reste aryle lié par l'intermédiaire d' un reste alkylène en $C_1$ à $C_6$,
ou bien $R^1$ et $R^2$ forment ensemble respectivement le groupe $OCH_2O$, $OCH_2CH_2O$, CH=CHO, $CH=C(CH_3)O$ ou CH=CHNH,
les restes $R^4$, $R^5$ identiques ou différents, représentent H ou un reste alkyle en $C_1$ à $C_6$,
les restes $R^6$, $R^7$, identiques ou différents, représentent H, un reste alkyle en $C_1$ à $C_6$, un reste aryle ou un reste aryle lié par l'intermédiaire d'un groupe alkylène en $C_1$ à $C_6$,
les restes alkyle pouvant également dans chaque cas être substitués au moins une fois, de façon identique ou différente, avec un halogène et/ou un groupe hydroxy, et
les restes aryle pouvant également représenter des restes phényle ou naphtyle substitués dans chaque cas au moins une fois avec un groupe OH, un halogène, un reste $CF_3$, un reste alkyle en $C_1$ à $C_6$, un reste alkoxy en $C_1$ à $C_6$, un reste cycloalkoxy en $C_1$ à $C_7$, un reste cycloalkyle en $C_3$ à $C_7$, un reste alkylène en $C_2$ à $C_6$ ou un reste phényle,
sous forme de sa base et/ou d'un sel d'acide acceptable du point de vue physiologique, et le cas échéant d'autres substances actives et/ou substances auxiliaires.

**18.** Médicament selon la revendication 17, contenant comme substance pharmaceutique active au moins un dérivé substitué de 2-aminoalkylbiphényle choisi dans le groupe comprenant les composés suivants :

(3'-méthoxybiphényl-2-ylméthyl)-diméthylamine et le chlorhydrate correspondant,
(4'-chlorobiphényl-2-ylméthyl)-diméthylamine et le chlorhydrate correspondant,
2'-diméthylaminométhylbiphényl-3-ol et le chlorhydrate correspondant,
(2'-méthoxybiphényl-2-ylméthyl)-diméthylamine et le chlorhydrate correspondant,
(3'-chlorobiphényl-2-ylméthyl)-diméthylamine et le chlorhydrate correspondant,
(2'-fluorobiphényl-2-ylméthyl)-diméthylamine et le chlorhydrate correspondant,
(3'-fluorobiphényl-2-ylméthyl)-diméthylamine et le chlorhydrate correspondant,
(4'-fluorobiphényl-2-ylméthyl)-diméthylamine et le chlorhydrate correspondant,
(3'-chloro-4'-fluorobiphényl-2-ylméthyl)-diméthylamine et le chlorhydrate correspondant,
(3'-méthoxybiphênyl-2-yléthyl)-diméthylamine et le chlorhydrate correspondant,
diméthyl-[2-(2-méthylbenzofuranne-4-yl)-benzyl]-amine et le chlorhydrate correspondant,
chlorhydrate de 2'-diméthylaminométhylbiphényl-2-carbaldéhyde,
(3'-difluorométhylbiphényl-2-ylméthyl)-diméthylamine et le chlorhydrate correspondant,
2'-diméthylaminométhylbiphényl-3-carbaldéhyde et le chlorhydrate correspondant,
chlorhydrate de biphényl-2-ylméthyldiméthylamine,
(3',4'-dichlorobiphényl-2-ylméthyl)-diméthylamine et le chlorhydrate correspondant,
(3',5'-dichlorobiphényl-2-ylméthyl)-diméthylamine et le chlorhydrate correspondant,
diméthyl-(4'-nitro-3'-trifluorométhylbiphényl-2-ylméthyl)-amine et le chlorhydrate correspondant,
(3',4'-difluorobiphényl-2-ylméthyl)-diméthylamine et le chlorhydrate correspondant,
(4'-fluoro-3'-trifluorométhylbiphényl-2-ylméthyl)-diméthylamine et le chlorhydrate correspondant,
(4'-chloro-3'-méthoxybiphényl-2-ylméthyl)-diméthylamine et le chlorhydrate correspondant,
N-(2'-diméthylaminométhyl-3-trifluorométhoxybiphényl-4-yl)-acétamide et le chlorhydrate correspondant,
(3'-isopropoxybiphényl-2-ylméthyl)-diméthylamine et le chlorhydrate correspondant,
2'-(2-diméthylaminoéthyl)biphényl-3-ol et le chlorhydrate correspondant,
4-chloro-2'-diméthylaminométhylbiphényl-3-ol et le chlorhydrate correspondant,
[2-(1*H*-indole-5-yl)benzyl)-diméthylamine et le chlorhydrate correspondant,
(4'-méthanesulfonylbiphényl-2-ylméthyl)-diméthylamine et le chlorhydrate correspondant,
(2',4'-dichlorobiphényl-2-ylméthyl)-diméthylamine et le chlorhydrate correspondant,
(2',3'-difluorobiphényl-2-ylméthyl)-diméthylamine et le chlorhydrate correspondant,
(2',5'-difluorobiphényl-2-ylméthyl)-diméthylamine et le chlorhydrate correspondant,
(2-benzo [1, 3] dioxol-5-ylbenzyl) -diméthylamine et le chlorhydrate correspondant,
1- [2'- (2-diméthylaminoéthyl) biphényl-3-yl] -éthanone et le chlorhydrate correspondant,
[2-(3',4'-diméthoxybiphényl-2-yléthyl]-diméthylamine et le chlorhydrate correspondant,
[2-(3'-isopropoxybiphényl-2-yl)éthyl]-diméthylamine et le chlorhydrate correspondant,
[2-(4'-chloro-3'-méthoxybiphényl-2-yl)éthyl]-diméthylamine et le chlorhydrate correspondant,
4-chloro-2'-(2-diméthylaminoëthyl)biphényl-3-ol et le chlorhydrate correspondant,
diméthyl-(3'-nitrobiphényl-2-ylméthyl) amine et le chlorhydrate correspondant,
4-amino-2'-diméthylaminométhylbiphényl-3-ol et le dichlorhydrate correspondant,
(3',5'-difluorobiphényl-2-ylméthyl)-diméthylamine et le chlorhydrate correspondant,
(2',5'-diméthoxybiphényl-2-ylméthyl)-diméthylamine et le chlorhydrate correspondant,

2'-diméthylaminométhyl-5-trifluorométhoxybiphényl-2-ylamine et le dichlorhydrate correspondant,
N-(2'-diméthylaminométhyl-5-trifluorométhoxybiphényl-2-yl)-acétamide et le chlorhydrate correspondant, ainsi que
3,5-dichloro-2'-diméthylaminométhyl-biphényl-4-ylamine et le chlorhydrate correspondant.

**19.** Médicament selon la revendication 17 ou 18, destiné à traiter / combattre des douleurs, des réactions inflammatoires et allergiques, des dépressions, l'abus de drogues et d'alcool, la gastrite, la diarrhée, l'incontinence d'urine, des maladies cardio-vasculaires, des maladies des voies respiratoires, la toux, des troubles psychiques et/ou l'épilepsie.

**20.** Utilisation d'au moins un dérivé substitué de 2-aminoalkylbiphényle de formule générale I suivant la revendication 17 sous forme de sa base et/ou d'un sel d'un acide acceptable du point de vue physiologique pour la préparation d'un médicament destiné à combattre des douleurs.

**21.** Utilisation d'au moins un dérivé substitué de 2-aminoalkylbiphényle de formule générale I selon la revendication 17 sous forme de ses bases et/ou d'un sel d'acides acceptables du point de vue physiologique pour la préparation d'un médicament destiné au traitement de réactions inflammatoires.

**22.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de réactions allergiques.

**23.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de dépressions.

**24.** Utilisation d'au moins un dérivé substitué de 2-aminoalkylbiphényle de formule générale I selon la revendication 17 sous forme de ses bases et/ou d'un sel d'acides acceptables du point de vue physiologique pour la préparation d'un médicament destiné au traitement de l'abus de drogues et/ou d'alcool.

**25.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de la gastrite.

**26.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de la diarrhée.

**27.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de l'incontinence d'urine.

**28.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de maladies cardio-vasculaires.

**29.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de maladies des voies respiratoires.

**30.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de la toux.

**31.** Utilisation d'au moins un dérivé substitué de 2-aminoalkylbiphényle de formule générale I selon la revendication 17 sous forme de sa base et/ou d'un sel d'un acide acceptable du point de vue physiologique pour la préparation d'un médicament destiné au traitement de troubles psychiques.

**32.** Utilisation d'au moins un dérivé substitué de 2-aminoalkylbiphényle de formule générale I selon la revendication 17

sous forme de sa base et/ou d'un sel d'un acide acceptable du point de vue physiologique pour la préparation d'un médicament destiné au traitement de l'épilepsie.

**33.** Utilisation selon l'une des revendications 20 à 32, **caractérisée en ce que** le dérivé substitué de 2-aminoalkylbiphényle est choisi dans le groupe comprenant les composés suivants:

(3'-méthoxybiphényl-2-ylméthyl)-diméthylamine et le chlorhydrate correspondant,
(4'-chlorobiphényl-2-ylméthyl)-diméthylamine et le chlorhydrate correspondant,
2'-diméthylaminométhylbiphényl-3-ol et le chlorhydrate correspondant,
(2'-méthoxybiphényl-2-ylméthyl)-diméthylamine et le chlorhydrate correspondant,
(3'-chlorobiphényl-2-ylméthyl)-diméthylamine et le chlorhydrate correspondant,
(2'-fluorobiphényl-2-ylméthyl)-diméthylamine et le chlorhydrate correspondant,
(3'-fluorobiphényl-2-ylméthyl)-diméthylamine et le chlorhydrate correspondant,
(4'-fluorobiphényl-2-ylméthyl)-diméthylamine et le chlorhydrate correspondant,
(3'-chloro-4'-fluorobiphényl-2-ylméthyl)-diméthylamine et le chlorhydrate correspondant,
(3'-méthoxybiphényl-2-yléthyl)-diméthylamine et le chlorhydrate correspondant,
diméthyl-[2-(2-méthylbenzofuranne-4-yl)-benzyl]-amine et le chlorhydrate correspondant,
chlorhydrate de 2'-diméthylaminométhylbiphényl-2-carbaldéhyde,
(3'-difluorométhylbiphényl-2-ylméthyl)-diméthylamine, et le chlorhydrate correspondant,
2'-diméthylaminométhylbiphényl-3-carbaldéhyde et le chlorhydrate correspondant,
chlorhydrate de biphényl-2-ylméthyldiméthylamine,
(3',4'-dichlorobiphényl-2-ylméthyl)-diméthylamine et le chlorhydrate correspondant,
(3',5'-dichlorobiphényl-2-ylméthyl)-diméthylamine et le chlorhydrate correspondant,
diméthyl-(4'-nitro-3'-trifluorométhylbiphényl-2-ylméthyl)-amine et le chlorhydrate correspondant,
(3',4'-difluorobiphényl-2-ylméthyl)-diméthylamine et le chlorhydrate correspondant,
(4'-fluoro-3'-trifluorométhylbiphényl-2-ylméthyl)-diméthylamine et le chlorhydrate correspondant,
(4'-chloro-3'-méthoxybiphényl-2-ylméthyl)-diméthylamine et le chlorhydrate correspondant,
N-(2'-diméthylaminométhyl-3-trifluorométhoxybiphényl-4-yl)-acétamide et le chlorhydrate correspondant,
(3'-isopropoxybiphényl-2-ylméthyl)-diméthylamine et le chlorhydrate correspondant,
2'-(2-diméthylaminoéthyl)biphényl-3-ol et le chlorhydrate correspondant,
4-chloro-2'-diméthylaminométhylbiphényl-3-ol et le chlorhydrate correspondant,
[2-(1*H*-indole-5-yl)benzyl]-diméthylamine et le chlorhydrate correspondant,
(4'-méthanesulfonylbiphényl-2-ylméthyl)-diméthylamine et le chlorhydrate correspondant,
(2',4'-dichlorobiphényl-2-ylméthyl)-diméthylamine et le chlorhydrate correspondant,
(2',3'-difluorobiphényl-2-ylméthyl)-diméthylamine et le chlorhydrate correspondant,
(2',5'-difluorobiphényl-2-ylméthyl)-diméthylamine et le chlorhydrate correspondant,
(2-benzo[1,3]dioxol-5-ylbenzyl)-diméthylamine et le chlorhydrate correspondant,
1-[2'-(2-diméthylaminoéthyl)biphényl-3-yl]-éthanone et le chlorhydrate correspondant,
[2-(3',4'-diméthoxybiphényl-2-yléthyl]-diméthylamine et le chlorhydrate correspondant,
[2-(3'-isopropoxybiphényl-2-yl)éthyl]-diméthylamine et le chlorhydrate correspondant,
[2-(4'-chloro-3'-méthoxybiphényl-2-yl)éthyl]-diméthylamine et le chlorhydrate correspondant,
4-chloro-2'-(2-diméthylaminoéthyl)biphényl-3-ol et le chlorhydrate correspondant,
diméthyl-(3'-nitrobiphényl-2-ylméthyl)amine et le chlorhydrate correspondant,
4-amino-2'-diméthylaminométhylbiphényl-3-ol et le chlorhydrate correspondant,
(3',5'-difluorobiphényl-2-ylméthyl)-diméthylamine et le chlorhydrate correspondant,
(2',5'-diméthoxybiphényl-2-ylméthyl)-diméthylamine et le chlorhydrate correspondant,
2'-diméthylaminométhyl-5-trifluorométhoxybiphényl-2-ylamine et le dichlorhydrate correspondant,
N-(2'-diméthylaminométhyl-5-trifluorométhoxybiphényl-2-yl)-acétamide et le chlorhydrate correspondant, ainsi que
3,5-dichloro-2'-diméthylaminométhyl-biphényl-4-ylamine et le chlorhydrate correspondant.